# EUROPEAN PATENT APPLICATION

(11) **EP 4 850 522 A1**
(43) Date of publication of application: **09.12.2026**
(21) Application number: 26200555.6
(22) Date of filing: 12.05.2021
(51) Int. Cl.: A01H 11/00, A01G 22/30

(54) **METHOD OF PROVIDING SEEDSTOCKS OF SPHAGNUM**

(30) Priority: 12.05.2020 GB 202006992; 12.05.2020 GB 202006991
(62) Document number(s) of the earlier application(s) in accordance with Art. 76 EPC: 25153292.5 / 4 520 167; 21727200.4 / 4 149 238
(71) Applicant: Micropropagation Services (E.M.) Limited, Loughborough, Leicestershire LE12 6NZ (GB)
(72) Inventor: WRIGHT, Neal, Loughborough, Leicestershire, LE12 6NZ (GB)
(74) Representative: Mathys & Squire

(57) **Abstract**

Disclosed herein is a method comprising providing *Sphagnum,* wherein the *Sphagnum* comprises *in vitro Sphagnum* that has been applied to a first growth surface, cultivated *in vivo* on the first growth surface, harvested from the first growth surface, and chopped to provide a seedstock of first generation *in vivo Sphagnum,* wherein the seedstock of first generation *in vivo Sphagnum* has been applied to a second growth surface and cultivated *in vivo* on the second growth surface, and has been harvested to provide the *Sphagnum;* and planting the *Sphagnum* at a site.

## Description

The present disclosure relates to *Sphagnum,* in particular to methods of providing a seedstock of *Sphagnum.*

*Sphagnum* is a genus of moss. It is a lower plant, or a non-vascular plant, and is an example of a bryophyte. It is often referred to as peat moss and typically grows in the wild in peatlands or wetlands. Examples of suitable habitats for *Sphagnum* include bogs, such as raised bogs and blanket bogs, moors, mires, and fens. *Sphagnum* has a particularly high capacity for maintaining water in its hyaline cells. As such, in its natural environment, *Sphagnum* typically grows in wet conditions such as in peatlands.

Peatlands around the world are formed when lower layers of *Sphagnum* decay to form peat, while the upper layer continues to grow on the surface. As a result of this, carbon is stored within the peat while the actively-growing upper *Sphagnum* continues sequestering carbon dioxide from the atmosphere. Peatlands cover approximately 3% of the land on the Earth's surface, but store over 500 Gigatonnes of carbon - more than all other vegetation types combined. However, due to adverse impacts on the peatlands (e.g. industrial pollution, drainage - particularly for agriculture, and peat harvesting) the actively-growing upper *Sphagnum* has been eroded (or is now absent) in many peatlands, thereby exposing the peat to the atmosphere. This absence of surface *Sphagnum* enables carbon to be released from the peatland. This is a pressing environmental issue, and damaged peatlands now contribute around 6% of global anthropogenic carbon dioxide emissions. As a result, there is a pressing need for effective peatland restoration and methods of effectively growing *Sphagnum* for restoration purposes. Conventional methods of peatland restoration typically involve translocating *Sphagnum* from other sites including peatlands, which is clearly not sustainable.

Peat is also used as horticultural growing media. As this peat is harvested from the wild, this damages peatlands and ultimately exacerbates carbon emissions. There is a growing demand for alternatives to peat in growing media, and *Sphagnum* itself has been identified as a key peat alternative. Therefore, there exists a need for an effective method of growing *Sphagnum* for the purposes of harvest, such as for growing media.

Unlike higher plants or vascular plants, *Sphagnum* does not have roots. This has several implications, one of which is that water is not primarily taken up from below (e.g. from roots), and instead water is absorbed through the main body of the plant, such as the stem, leaves, or branches. *Sphagnum* spreads primarily from new offshoots called innovations. As used herein, the term "innovation" may otherwise be referred to as a "growing point". An individual plant of *Sphagnum* grows in a strand. A strand of *Sphagnum* comprises a stem with small leaves arranged intermittently along the length of the stem. The strand also comprises larger branches shooting off from the stem. At the top of the stem, the strand comprises a capitulum which is a head of new innovations. The capitulum is the primary growth point of *Sphagnum.* Due to this, water is primarily desired at the top of *Sphagnum,* which makes good use of collecting water e.g. from rain or irrigation from above. *Sphagnum* can also comprise innovations where branches sprout off from the main stem. The innovations can eventually result in new stems which break off from the original stem to form a new strand of *Sphagnum.* Multiple strands of *Sphagnum* together grow as a hummock. The creation of new strands is a result of vegetative reproduction of *Sphagnum,* and allows *Sphagnum* to grow into carpets covering a surface. *Sphagnum* can also reproduce via spores, but the abundance of spores of *Sphagnum* is often very low in the wild, and at most sites reproduction via spores is limited, and in any case is limited to a specific and narrow window of time in the year. As such, vegetative reproduction often dominates.

Another property of *Sphagnum* resulting from its lack of roots is that it cannot be planted in conventional ways. Typical methods of applying *Sphagnum* to a surface involve partially burying a clump of *Sphagnum,* such as in undergrowth on a peatland. Providing clumps of *Sphagnum* results in a non-uniform coverage of *Sphagnum* in the discrete areas of the clumps, and requires a large amount of starting material which can be expensive, especially if a high degree of initial coverage is desired. As *Sphagnum* is often translocated from other wild sources, using a large amount of starting material is of course damaging to the environment. Where a sustainable production of *Sphagnum* is possible, for example through tissue culture, the damage to the environment can be mitigated, but the cost of providing an initial large amount of starting material can be expensive, and often prohibitively so.

Other methods of applying *Sphagnum* include dispersing strands of *Sphagnum* onto a surface, such as an exposed peatland for restoration. Such methods are ineffective as the strands are not secured to the ground, often dry out before establishing and anchoring themselves, and blow away in wind or rain - both of which are particularly significant factors on peatlands.

The present disclosure seeks to address one or more of the above problems.

Aspects of the invention are set out in the independent claims and preferred features are set out in the dependent claims.

According to a first aspect of the present disclosure, there is provided a suspension of *Sphagnum,* comprising: a fluid solution comprising: water; and a thickening agent dissolved in the water, wherein the thickening agent comprises a cellulose-based or a starch-based thickening agent; and a plurality of fragments of *Sphagnum* suspended in the solution.

Disclosed herein is a suspension of *Sphagnum,* comprising: a fluid solution comprising: water; and a cellulose-based thickening agent dissolved in the water; and a plurality of fragments of *Sphagnum* suspended in the solution.

Disclosed herein is a suspension of *Sphagnum,* comprising: a fluid solution comprising: water; a thickening agent; and a plurality of fragments of *Sphagnum* suspended in the solution. Optionally, the suspension further comprises nutrients. Optionally, the nutrients comprise calcium. Optionally, the thickening agent comprises a cellulose-based thickening agent. Optionally, the thickening agent comprises a starch-based thickening agent.

The inventors have developed the suspension of the present disclosure which provides a medium for carrying *Sphagnum* to allow effective spreading of *Sphagnum* onto a surface, for example onto a peatland for restoration or onto a surface for propagation, e.g. in a greenhouse or on a field. Optionally, the suspension is suitable for coating a surface, in particular a growth surface on which *Sphagnum* can be grown. Optionally, the *Sphagnum* in the suspension is suitable for growth. In other words, it is not dried out or otherwise dead. Preferably, the suspension is not toxic to the *Sphagnum.*

The suspension comprises a fluid solution. The fluid solution comprises water. Preferably, the water may be rainwater, or other low-salt water such as distilled water, or reverse osmosis water. In other examples, the water may be deionised water or otherwise clean water. Due to the presence of the water, the solution may be referred to as an aqueous solution. The fluid solution is preferably a liquid at room temperature, in particular at 20 °C. Preferably, the fluid solution is a liquid above 0 °C and below 50 °C, preferably at least between 5 °C and 25 °C.

The fluid solution also comprises a thickening agent. The thickening agent is dissolved into the water, and is thus water-soluble. The thickening agent acts to thicken the solution. In other words, the thickening agent increases the viscosity of the solution. The viscosity of the solution is thus higher than liquid water alone. This solution may be referred to as a thickened fluid solution or a viscous fluid. Optionally, the fluid solution may be referred to as a liquid gel.

The fluid solution should be differentiated from solids in which sufficient amounts of thickening agents, gelling agents, or solidifying agents are added to a liquid such that the liquid turns into a solid. In other words, the fluid solution is not a solid. In one example, the fluid solution is not a solid gel. In contrast, the fluid solution is able to flow. In particular, the fluid solution may be able to take on the shape of its container, for example as a liquid.

The thickening agent is dissolved in the water. This should be differentiated from situations where a thickening agent is merely mixed with water. One such example is where the thickening agent comprises a starch-based thickening agent. Starch is insoluble in water at room temperature. Therefore, merely adding starch to water will not provide a thickened fluid solution as described herein. Instead, the starch can be heated to dissolve into the water in a process referred to as starch gelatinization, at which point the starch acts to thicken the water to form a fluid solution.

In the first aspect of the present disclosure, the thickening agent comprises a cellulose-based or a starch-based thickening agent. As used herein, "cellulose-based" preferably connotes a thickening agent comprising cellulose, such as a compound containing cellulose. For example, the thickening agent contains cellulose and is made from cellulose, but may be formed of other atoms or molecules. In other words, the thickening agent may be derived from cellulose. One such example of a cellulose-based thickening agent is hydroxyethyl cellulose. Equally, "starch-based" preferably connotes a thickening agent comprising starch.

In some examples, the suspension contains one or more thickening agents. For example, the suspension may contain a cellulose-based thickening agent and a starch-based thickening agent. In other examples, the thickening agent may consist of a particular thickening agent. For example, the thickening agent may consist of a cellulose-based thickening agent. For example, the thickening agent may consist of a starch-based thickening agent.

Preferably, the thickening agent comprises a cellulose-based thickening agent. The cellulose-based thickening agent is more preferable to the starch-based thickening agent. Cellulose is a polysaccharide. It is preferable for the cellulose to be obtained from plant material.

More preferably, the thickening agent comprises a cellulose ether. Even more preferably, the thickening agent comprises hydroxyethyl cellulose. Hydroxyethyl cellulose is often referred to as HEC. Hydroxyethyl cellulose has been found to be particularly preferable as it is compatible with *Sphagnum,* and has been found to be non-toxic and to support growth of the *Sphagnum.* It has also been found to provide the desired physical characteristics of the fluid solution, including the ability to suspend *Sphagnum.*

Preferably, the hydroxyethyl cellulose has an average molecular weight of at least 500,000 Da (daltons, or unified atomic mass unit), more preferably at least 750,000 Da, even more preferably at least 1,000,000 Da, still more preferably at least 1,250,000 Da. In one example, the average molecular weight is between 1,000,000 Da and 1,500,000 Da. Providing a higher average molecular weight allows the viscosity to be higher for a given concentration in aqueous solution.

For example, the thickening agent may be Natrosol^{™} hydroxyethyl cellulose, commercially available in powder form from Ashland, USA. In some examples, the thickening agent may comprise Natrosol^{™} 250 HHW. More preferably, the thickening agent comprises Natrosol^{™} 250 HX or HHX.

Natrosol^{™} 250 HX has an average molecular weight of 1,000,000 Da, Natrosol^{™} 250 HHX has an average molecular weight of 1,300,000 Da, and Natrosol^{™} 250 HHW has an average molecular weight of 1,300,000 Da. Most preferably, the thickening agent comprises Natrosol^{™} 250 HHX.

Hydroxyethyl cellulose can be dissolved in water at room temperature in order to thicken the fluid solution. As such, it has been found to be a particularly preferable thickening agent.

Optionally, the thickening agent comprises methyl cellulose, such as carboxymethyl cellulose.

Optionally, the fluid solution is viscoelastic. This means that it exhibits both viscous and elastic properties. Optionally, the fluid solution is a shear-thinning fluid. Optionally, the fluid solution is a non-Newtonian fluid. An aqueous solution of hydroxyethyl cellulose is an example of a viscoelastic, shear-thinning, and non-Newtonian fluid.

Optionally, the fluid solution comprises between 1 g and 20 g of hydroxyethyl cellulose per L of water, more preferably between 2 g and 18 g, even more preferably between 3 g and 15 g, and still more preferably between 4 g and 12 g. Even more preferably, the fluid solution comprises between 5 g and 10 g of hydroxyethyl cellulose per L of water. This has been found to provide an optimum viscosity to provide the benefits as described herein. For Natrosol^{™} HX, preferably the solution comprises between 5 g and 15 g of thickening agent per L of water, more preferably between 8 g and 10 g, most preferably about 9 g per L. For Natrosol^{™} HHX, preferably the solution comprises between 2 g and 10 g of thickening agent per L of water, more preferably between 5 g and 8 g. For example, there may be between 5 g and 10 g per L. In one particularly preferred example, there is 7.25 g per L of HHX. At around 20 °C, this provides optimum properties of the suspension, such as the viscosity. For example, providing between 5 and 15 g of hydroxyethyl cellulose per L of water ensures that the *Sphagnum* is well suspended and flows with the fluid solution and does not float or sink when stored in containers.

In a particularly preferred embodiment, the fluid solution comprises between 2 g and 13 g of hydroxyethyl cellulose per L of water, wherein the hydroxyethyl cellulose has an average molecular weight of between 1,000,000 Da and 1,300,000 Da. More preferably, the fluid solution with the hydroxyethyl cellulose having an average molecular weight of between 1,000,000 Da and 1,300,000 Da comprises between 3 g and 12 g of hydroxyethyl cellulose per L of water, even more preferably between 4 g and 11 g, and still more preferably between 5 g and 10 g.

Preferably, the ratio of an average molecular weight of the hydroxyethyl cellulose (Da) to a weight of hydroxyethyl cellulose per L of water is between 50,000 and 500,000, more preferably between 75,000 and 350,000, even more preferably between 100,000 and 200,000.

Preferably, the thickening agent comprises a starch-based thickening agent. Starch is a polysaccharide. It is preferable for the starch to be obtained from plant material. For example, starch in the form of cornflour is known to be a thickening agent. It is more preferable for the thickening agent to comprise a cellulose-based thickening agent than starch because this has been found to better support growth of *Sphagnum* without causing damage.

As mentioned above, starch can be dissolved in water by heating the starch and water. Depending on the starch, the mixture can be heated to around 55 °C - 85 °C in order to dissolve the starch in the water. This is known as starch gelatinization. By dissolving starch in water in this manner, a thickened fluid solution can be obtained.

Optionally, the fluid solution comprises between 10 g and 100 g of starch per L of water, preferably between 20 g and 50 g, more preferably between 30 g and 40 g. Optionally, the thickening agent comprises a natural starch, such as a maize starch. Optionally, the thickening agent comprises a partially pregelatinized starch. Preferably, the thickening agent comprises between 30 g and 40 g of a partially pregelatinized starch. Optionally, the thickening agent comprises LYCATAB (RTM) PGS, commercially available from Roquette Pharma. Preferably, the thickening agent comprises a pregelatinized starch (i.e. fully pregelatinized). This is soluble is cold water, which is preferable for providing the thickening agent dissolved in water. Optionally, the thickening agent comprises PREGEFLO (RTM) CH 40, commercially available from Roquette Pharma.

Optionally, the thickening agent comprises an extract from a plant. Preferably, the thickening agent comprises an extract from a vascular plant. Vascular plants have conductive (vascular) tissues such as a xylem or phloem, for transporting resources. A vascular plant may otherwise be referred to as a higher plant. Preferably, the term vascular plant excludes non-vascular plants (i.e. lower plants) such as bryophytes and algae. As such, the thickening agent can be made from a plant-based extract from a vascular plant. For example, the plant-based extract may be cellulose or starch.

Optionally, the thickening agent comprises a polymer-based thickening agent. A polymer is a molecule of repeating monomer sub-units. The polymer may be natural or synthetic.

Optionally, the thickening agent comprises a polysaccharide-based thickening agent. A polysaccharide is a carbohydrate polymer chain comprising monomers of monosaccharides bonded together. Starch is an example of a polysaccharide, and is made from a chain of glucose monomers, where glucose is a monosaccharide. A polysaccharide-based thickening agent preferably means a thickening agent derived from or comprising a polysaccharide.

Optionally, the thickening agent comprises a protein-based thickening agent. For example, the thickening agent may comprise gelatine. In another example, the thickening agent may comprise collagen. Optionally, the thickening agent may comprise chickpea water.

Optionally, the thickening agent may comprise guar gum. Optionally, the thickening agent may comprise xanthan gum. Optionally, the thickening agent comprises a fibre-based thickening agent. Optionally, the thickening agent may comprise chia seeds. Optionally, the thickening agent comprises psyllium husk. Optionally, the thickening agent comprises a flour for example coconut flour, chickpea flour, or cornflour.

Optionally, the thickening agent does not comprise sodium alginate. Optionally, the thickening agent does not comprise algin. Algin, or otherwise known as alginic acid, is a polysaccharide. Algin is derived from algae (a non-vascular plant), and is thus not derived from a vascular plant. Algin is often used in salt form as sodium alginate. Sodium alginate is soluble in water, and is often combined with calcium, such as in the form of calcium chloride, to solidify the sodium alginate solution into a solid gel. The calcium forms cross links with the alginate and causes the solution to solidify, and the resultant calcium alginate is not soluble in water. This method is often used for producing solid alginate beads, such as in food preparation. Such a result is not preferable for the suspension of the present disclosure, as this would inhibit the beneficial properties of the fluid solution. In particular, the solid alginate gel would not be a fluid, and would instead set into a solid. In other words, the solid alginate gel would set, and would not be non-setting. Using sodium alginate and calcium chloride would therefore cause setting of the gel, and has been found to form undesirable clumps around the *Sphagnum.* This prevents a suspension comprising a fluid solution because instead the gel solidifies, meaning the *Sphagnum* cannot be spread as desired herein.

Moreover, it is preferable to not use sodium alginate in the solution of the present disclosure because the algin clumps and solidifies in the presence of calcium nutrients. In some cases, it may be desirable to add calcium as a nutrient in the suspension, as described in more detail below, in which case sodium alginate would not be desirable for a thickening agent. In some cases, the water used may comprise nutrients such as calcium (such as in ground water which absorbs calcium from soil/rocks), and therefore alginate may solidify when used even where calcium is not specifically added as a nutrient. Furthermore, calcium can often be found in soil where the suspension is spread, and may cause the alginate to solidify. Additionally, alginate has been found to impede growth of *Sphagnum* as alginate clumps around the *Sphagnum* and prevents water and air uptake.

Generally, the algin would also react to solidify the solution to form a solid gel in the presence of any divalent cation. Divalent means having a valence of 2. For example, the divalent cation may comprise calcium (i.e. Ca²⁺). In other examples, the divalent cation may comprise magnesium. Other divalent cations include iron (2+), beryllium, strontium, barium, and radium.

It is therefore desirable to select a thickening agent which retains a fluid solution, even in the presence of calcium. A cellulose-based thickening agent or a starch-based thickening agent has been found to be preferable.

Preferably, the thickening agent does not comprise agar. Agar is a solid at room temperature (e.g. 20 °C), and therefore does not provide a fluid solution suitable for use in the present disclosure. Although agar can be used as a solid growth medium, such as during *in vitro* cultivation of *Sphagnum,* this does not form a suspension as the *Sphagnum* merely rests on the solid medium, and the agar does not form a fluid solution.

In cases where the thickening agent is a polymer, the thickening agent optionally comprises a homopolymer. A homopolymer is a polymer comprising repeating monomer units of the same monomer. This is in contrast, for example, to a copolymer which comprises two or more monomers alternating in a chain. Optionally, the homopolymer comprises a glucose monomer. For example, cellulose and starch are homopolymers comprising glucose monomers. As an example, algin is a copolymer and is not a homopolymer.

In cases where the thickening agent is a polymer, each monomer of the thickening agent is optionally electrically neutral. Optionally, each monomer of the thickening agent does not comprise a carboxylate group. For example, cellulose and starch have glucose monomers which are electrically neutral and do not comprise a carboxylate group.

Optionally, the fluid solution also comprises nutrients. The nutrients can be provided to facilitate growth of the *Sphagnum* within the suspension. The nutrients can be dissolved into the water, and are thus water-soluble. For example, the nutrients may be in the form of nutritional salts. The nutrients can provide conditions which facilitate the growth of *Sphagnum.* This can be particularly beneficial for fragments of *Sphagnum* when they initially begin growing. Especially on peatlands where conditions are harsh, providing nutrients to facilitate growth can result in better establishment, especially initially. By providing the nutrients in the suspension, it is not necessary to apply any further nutrients for a set period of time, for example until after the *Sphagnum* has established and starts growing rapidly (e.g. two to three weeks when grown in a greenhouse, or two to three months when grown in a field). When *Sphagnum* is grown on a peatland, it is not normally supplemented with nutrients after establishment due to its inaccessibility and therefore providing the initial nutrient supply is especially beneficial. Where *Sphagnum* is grown for growing media purposes, such as in a greenhouse or in a field, it may be beneficial to treat the *Sphagnum* at a later stage with irrigation and further nutrients to facilitate growth. In such cases, the nutrients in the suspension provide an initial source.

The nutrients can be mixed throughout the solution leading to uniform access to the nutrients by the *Sphagnum.* Optionally, the nutrients are homogeneously or substantially homogeneously dispersed throughout the suspension. This facilitates uniformity of growth of the *Sphagnum* when the suspension is applied over a surface, due to the uniform supply of nutrients.

The thickened solution also retains the nutrients within the suspension. In the absence of a thickening agent, the nutrients would not be well retained, and could be washed away or separated from the *Sphagnum.* The thickening agent therefore holds the nutrients to provide a supply to the *Sphagnum* over an extended period of time. Because the nutrients are held in the suspension, this provides an improved system compared to simply providing nutrients via irrigation onto a suspension not comprising nutrients. Applying nutrients, particularly shortly after application of the suspension, can result in washing away the suspension before it is established. It is therefore preferable to not apply irrigation or treatment with nutrients for a period, such as at least two weeks, after application. However, this will depend on the environmental conditions, as for example if it is particularly dry then it may be desirable to apply a small amount of irrigation shortly after application to prevent desiccation of the *Sphagnum,* but avoid washing away the nutrient. Therefore, providing a source of nutrients can provide an initial supply where it is desirable not to apply nutrients for a period of time after application. Moreover, the nutrient can be absorbed by the *Sphagnum* and held loosely until required, which can be assisted by avoiding excess irrigation.

Optionally, the nutrients comprise calcium. Calcium has been found to be a particularly useful nutrient for the growth of *Sphagnum.* Preferably, the nutrients comprise at least 1 mg of calcium per L (litre) of water. Preferably, the nutrients comprise less than 100 mg of calcium per L of water. Preferably, the nutrients comprise between 1.17 mg and 92.39 mg of calcium per L of water. More preferably, the nutrients comprise between 1 mg and 50 mg of calcium per L of water, even more preferably between 2 mg and 25 mg, yet even more preferably between 5 mg and 20 mg. For example, the calcium may be provided in the form of a calcium salt, such as calcium oxide (CaO), calcium chloride (CaCl₂), or calcium nitrate (Ca(NO₃)₂). It has been found that even low levels of calcium can be beneficial to growth of *Sphagnum.*

The amount of nutrients can be adjusted depending on the amount of *Sphagnum* in the suspension. For example, the above nutrient levels may be used for 100 g of *Sphagnum* per L of suspension (100 g calculated using the standardised mass per volume by compressing *Sphagnum* by 16 g / cm², which provides a dry weight of around 1.43 g). It will be realised the values disclosed herein are optimised for 100 g of standard weight of *Sphagnum,* and each value may be scaled to a different concentration for a different amount of *Sphagnum.* For example, the nutrients may comprise between 1 mg and 100 mg of calcium per 100 g standardised weight (compressed by 16 g / cm²) of *Sphagnum.* Optionally, the nutrients may comprise between 0.7 mg and 70 mg of calcium per 1 g dry weight of *Sphagnum.*

Optionally, the suspension does not solidify in the presence of calcium. In other words, the fluid solution remains fluid in the presence of calcium. For example, calcium may be added to the suspension as a nutrient. In other cases, calcium may be present in the soil to which the suspension is applied. In yet other cases, calcium may be present in the water of the suspension, or water added to the suspension such as irrigation during growth. Therefore, it is preferable for the suspension to remain fluid and not solidify in the presence of calcium, which allows it to be adapted for use in cultivation. Optionally, the suspension does not solidify in the presence of any nutrients, including the nutrients disclosed herein such as magnesium.

Optionally, the nutrients comprise at least one of: magnesium, nitrogen, potassium, and/or phosphorus. For example, this can be in addition or instead of the presence of calcium.

Optionally, the nutrients comprise magnesium. Preferably, the nutrients comprise at least 0.1 mg of magnesium per L of water. Preferably, the nutrients comprise less than 50 mg of magnesium per L of water. Preferably, the nutrients comprise between 0.33 mg and 32.93 mg of magnesium per L of water. More preferably, the nutrients comprise between 1 mg and 15 mg of magnesium per L of water, even more preferably between 3 mg and 10 mg. As above, the amount of magnesium can be adjusted depending on the amount of *Sphagnum* per L of suspension. Optionally, the nutrients comprise between 0.1 mg and 50 mg of magnesium per 100 g standardised weight (compressed by 16 g / cm²) of *Sphagnum.* Optionally, the nutrients may comprise between 0.07 mg and 50 mg of magnesium per 1 g dry weight of *Sphagnum.*

Optionally, the nutrients comprise nitrogen. As used herein, the term "nitrogen" preferably includes nitrogen-containing elements or compounds, and is thus a total nitrogen content of the nutrients present. Preferably, "nitrogen" includes nitrate, ammonium, and ureic nitrogen.

Preferably, the nutrients comprise at least 15 mg of nitrogen per L of water. Preferably, the nutrients comprise less than 250 mg of nitrogen per L of water. Preferably, the nutrients comprise between 18.05 mg and 240.81 mg of nitrogen per L of water. More preferably, the nutrients comprise between 15 mg and 100 mg of nitrogen per L of water, even more preferably between 25 mg and 75 mg. As above, the amount of nitrogen can be adjusted depending on the amount of *Sphagnum* per L of suspension. Optionally, the nutrients comprise between 15 mg and 250 mg of nitrogen per 100 g standardised weight (compressed by 16 g / cm²) of *Sphagnum.* Optionally, the nutrients may comprise between 10 mg and 175 mg of nitrogen per 1 g dry weight of *Sphagnum.*

Optionally, the nutrients comprise phosphorus. Preferably, the nutrients comprise at least 5 mg of phosphorus per L of water. Preferably, the nutrients comprise less than 75 mg of phosphorus per L of water. Preferably, the nutrients comprise between 10.99 mg and 61.41 mg of phosphorus per L of water. More preferably, the nutrients comprise between 5 mg and 50 mg of phosphorus per L of water, even more preferably between 10 mg and 20 mg. As above, the amount of phosphorus can be adjusted depending on the amount of *Sphagnum* per L of suspension. Optionally, the nutrients comprise between 5 mg and 75 mg of phosphorus per 100 g standardised weight (compressed by 16 g / cm²) of *Sphagnum.* Optionally, the nutrients may comprise between 3.5 mg and 55 mg of phosphorus per 1 g dry weight of *Sphagnum.*

Optionally, the nutrients comprise potassium. Preferably, the nutrients comprise at least 20 mg of potassium per L of water. Preferably, the nutrients comprise less than 300 mg of potassium per L of water. Preferably, the nutrients comprise between 66.84 mg and 266.25 mg of potassium per L of water. More preferably, the nutrients comprise between 50 mg and 150 mg of potassium per L of water. As above, the amount of potassium can be adjusted depending on the amount of *Sphagnum* per L of suspension. Optionally, the nutrients comprise between 20 mg and 300 mg of potassium per 100 g standardised weight (compressed by 16 g / cm²) of *Sphagnum.* Optionally, the nutrients may comprise between 14 mg and 210 mg of potassium per 1 g dry weight of *Sphagnum.* The scaling of the nutrients may be applied to other values for each nutrient disclosed herein, wherein each disclosed value corresponds to 100 g of *Sphagnum* by standardised weight, or 1.43 g by dry weight.

Optionally, the nutrients comprise sulphur. Optionally, the nutrients comprise between 4.30 mg and 65.59 mg of sulphur per L of water.

Optionally, the nutrients comprise iron. Optionally, the nutrients comprise between 0.31 mg and 9.15 mg of iron per L of water.

Optionally, the nutrients comprise sodium, manganese, copper, zinc, boron, molybdenum, and/or chloride. Optionally, the nutrients comprise between 2.51 mg and 53.47 mg of sodium per L of water, between 0.21 mg and 1.94 mg of manganese per L of water, between 0.09 mg and 0.25 mg of copper per L of water, between 0.37 mg and 1.56 mg of zinc per L of water, between 0.14 mg and 1.02 mg of boron per L of water, between 0.01 mg and 0.15 mg of molybdenum per L of water, and/or between 0.16 mg and 97.64 mg of chloride per L of water.

Some nutrients may be present in the soil on which the suspension is spread, and therefore some nutrients may not be necessary to include in the suspension.

Optionally, the suspension does not comprise sugar (e.g. sucrose), vitamins, and/or plant growth hormones.

Optionally, the fluid solution does not solidify for at least 6 hours at a temperature between 5 °C and 25 °C. Preferably, the suspension is non-setting. As used herein, the term "non-setting" preferably means the suspension does not solidify for at least 6 hours, in particular at 20 °C, preferably between 5 °C and 25 °C. Furthermore, preferably the suspension is non-setting in the presence of calcium. In other words, preferably the fluid solution remains a liquid.

The fluid solution generally increases in thickness at a lower temperature but will not solidify until frozen. When a cellulose-based thickening agent such as hydroxyethyl cellulose is used, the solution does not freeze even at 0 °C. At higher temperatures, the solution will become less viscous and above 30 °C it will become very liquid. Above 40 °C it will start to damage the *Sphagnum.* It is therefore preferable to keep the suspension stored between 0 °C and 30 °C.

As the fragments of *Sphagnum* are suspended in the solution, the suspension is thus a thickened fluid solution carrying fragments of *Sphagnum,* which are held in the suspension.

Any suitable *Sphagnum* species (or optionally a combination thereof) may be used in the present disclosure. As different species of *Sphagnum* may have different growth requirements, the *Sphagnum* species for use in the present disclosure may be selected depending on the environment.

The suspension comprises one or more *Sphagnum* species. Any species could be used, but in one example the present disclosure comprises the use of one or more *Sphagnum* species selected from the group consisting of: *Sphagnum angustifolium, Sphagnum australe, Sphagnum capillifolium, Sphagnum centrale, Sphagnum compactum, Sphagnum cuspidatum, Sphagnum denticulatum, Sphagnum fallax, Sphagnum fimbriatum, Sphagnum fuscum, Sphagnum imbricatum (austinii), Sphagnum inundatum, Sphagnum magellanicum (medium), Sphagnum palustre, Sphagnum papillosum, Sphagnum pulchrum, Sphagnum russowii, Sphagnum squarrosum, Sphagnum subnitens, Sphagnum tenellum,* and *Sphagnum cristatum.* In one example, the method comprises the use of one or more *Sphagnum* species selected from the group consisting of: *Sphagnum palustre, Sphagnum capillifolium, Sphagnum capillifolium rubellum, Sphagnum subnitens, Sphagnum denticulatum, Sphagnum squarrosum, Sphagnum fallax, Sphagnum fimbriatum, Sphagnum cuspidatum, Sphagnum magellanicum,* and *Sphagnum papillosum.* In one example, the invention comprises the use of one or more *Sphagnum* species selected from the group consisting of: *Sphagnum palustre, Sphagnum capillifolium, Sphagnum capillifolium rubellum, Sphagnum subnitens, Sphagnum squarrosum, Sphagnum magellanicum,* and *Sphagnum papillosum.*

In one example, a *Sphagnum* species for use in the present disclosure may be one or more selected from the group consisting of: *Sphagnum palustre, Sphagnum capillifolium, Sphagnum* fallax, *Sphagnum magellanicum, Sphagnum papillosum,* and *Sphagnum squarrosum.*

Most preferably the *Sphagnum* species is *Sphagnum palustre.* For example, *Sphagnum palustre* may be preferable for use in a growing medium because of its physical properties.

It is also envisaged that the invention could be applied to any hybrid *Sphagnum* species.

Optionally, the plurality of fragments of *Sphagnum* comprises at least one of the *Sphagnum* species disclosed herein.

Optionally, the plurality of fragments of *Sphagnum* comprises at least 2, 3, 4, 5 or more *Sphagnum* species.

As the plurality of fragments of *Sphagnum* are suspended in the solution, preferably this means that the fragments of *Sphagnum* are held dispersed within the solution, and at least some of the fragments of *Sphagnum* do not float on the solution and/or do not sink beneath the solution. In other words, the fragments are not left behind when the solution is moved, such as by being poured or spread. This means that the *Sphagnum* can be distributed onto a surface effectively when the suspension is applied. Once the suspension has been applied, the suspension also retains the *Sphagnum* substantially in position such that the *Sphagnum* does not fall out of the suspension e.g. by sinking to the bottom. This enables the *Sphagnum* to be generally fixed in position with the suspension, and not for example to be washed away while the rest of the suspension is left behind. This also allows the *Sphagnum* to absorb the nutrients in the suspension over an extended period of time, which is particularly advantageous for the initial period of growth after application, where nutrient supply can aid establishment.

Optionally, the fragments of *Sphagnum* are dispersed evenly throughout the suspension. For example, the *Sphagnum* may be homogeneously spread out in the solution (i.e. uniform density). In some cases, it can be acceptable to provide a thoroughly mixed suspension, for example such that at least 50% of the fragments are not settled at the bottom or the top of the mixture.

The thickening agent provides properties of the suspension that help retain the *Sphagnum* itself within the suspension. By thoroughly mixing the *Sphagnum* within the solution of the suspension, the *Sphagnum* can be distributed throughout the suspension, and the viscosity of the solution will retain the *Sphagnum.* In the absence of a suitable thickening agent, the *Sphagnum* would not be held suspended by the water alone, and instead would sink to the bottom of the solution. Equally, the thickening agent and quantity thereof is preferably chosen to enable sufficient mixing. If the viscosity is too high, the *Sphagnum* will not mix in well, may float on top of the upper surface of the thick solution or may be damaged by the mixing.

Preferably, the suspension does not clump around the *Sphagnum.* By suitable choice of the thickening agent as described herein, the suspension will not clump around the *Sphagnum* and/or around the nutrients.

A cellulose-based thickening agent or a starch-based thickening agent acts as a suitable thickening agent which provides the above benefits.

The suspension can be used to provide a more uniform density of *Sphagnum* when spread over a surface than conventional techniques. In particular, the *Sphagnum* can be mixed to create a substantially uniform density of *Sphagnum* within the suspension and, because the suspension can retain the *Sphagnum* well, when the suspension is applied to the surface, the density can be preserved. The thickening agent aids this retention when compared to thinner, less viscous liquids such as in the absence of a thickening agent dissolved in the fluid solution.

The suspension can provide a uniform density of *Sphagnum* which can be applied more evenly than conventional techniques. For example, a substantially uniform density of *Sphagnum* can be applied by spraying, compared to planting individual clumps of *Sphagnum* over an area. Moreover, the coverage of a surface can be increased more easily, as the suspension can be spread to entirely cover a surface. The density of *Sphagnum* within the suspension can then be controlled to provide the desired initial coverage of *Sphagnum* over the area.

The thickened solution is preferably sticky. This allows the suspension to stick to the surface to which it is applied and provides a securing means to secure the suspension to the surface. This can alleviate the problem of unsecured *Sphagnum* blowing away or being washed away by rain. Instead, the suspension can retain the *Sphagnum* in position for long enough to provide nutrients to the *Sphagnum* during its initial establishment phase and long enough that the *Sphagnum* becomes established enough to continue growing.

Optionally, the suspension is adhesive to a growing substrate. Optionally, the suspension is adhesive to a growing substrate comprising soil, sand, compost, peat, and/or dried *Sphagnum.* This means the suspension sticks to the substrate when it is put into contact with it. For example, the substrate may be a peat surface or compost in a tray. This allows the suspension to retain the *Sphagnum* on the surface and anchor it.

Optionally, the suspension provides capillary contact with a surface to which it is applied to enable fluid transfer between the surface and the suspension. As used herein "capillary contact" preferably means providing a fluid pathway, such as through capillary action. In this manner, the suspension can contact the surface to which it is applied, and provide a fluid pathway between the surface and the *Sphagnum,* thereby enabling fluid transfer. For example, when applied to a peat surface, the suspension can enable the transfer of water and nutrients from the peat into the solution for use by the *Sphagnum* in growth. The capillary contact is promoted by the thickened fluid solution because the surface area in contact with the surface is increased compared to a solid (e.g. a solid gel) due to its flowing properties. The thickened suspension also retains that contact over time, which is advantageous compared to liquid without an appropriate thickening agent, which instead would wash or drain away. A cellulose-based or a starch-based thickening agent has been found to provide optimum capillary contact and promote fluid transfer. Using alternative thickening agents such as alginate has been found to inhibit fluid transfer and prevent air and water uptake.

The fluid solution in the suspension allows the suspension to be spread over a surface, for example by spraying. This allows vast areas to be covered quickly, especially when the spraying can be conducted by a machine or a hand-held sprayer, and much faster than applying clumps of *Sphagnum* by hand. Spraying also allows for much easier application than a solid suspension.

By providing fragments of *Sphagnum,* the *Sphagnum* can be sprayed more easily than long strands which have been found to clog and tangle in a spraying system. Preferably, by having fragments of length less than 50 mm, more preferably between 5 mm and 30 mm, the fragments are easily sprayed and avoid clogging or tangling.

Optionally, the suspension is capable of being sprayed through a nozzle having a diameter of between 5 mm and 10 mm. Thus, the viscosity of the suspension is provided to enable such spraying. If the suspension is too viscous, for example if it comprises a more solid gel, the suspension would not be able to be sprayed through such a nozzle. It is preferable for the suspension to be capable of being sprayed in this way, while retaining the *Sphagnum* dispersed in the solution. This provides an ideal viscosity, where if the viscosity is too high such as with a solid gel, the suspension could not be sprayed, and where if the viscosity is too low such as with a non-thickened solution, the suspension could not retain the *Sphagnum* and the *Sphagnum* would not be carried with the solution. Therefore, the thickened fluid solution of the present disclosure provides an optimised suspension for holding *Sphagnum* for applying to a surface.

Optionally, the fluid solution has a viscosity of between 1000 and 4000 mPa·s at 25 °C. More preferably, the fluid solution has a viscosity of between 1500 and 4000 mPa·s at 25 °C, even more preferably between 1750 and 3750, still more preferably between 2000 and 3500. As used herein, the viscosity is measured using the standard Brookfield scale, such as using a Brookfield Dial Reading Viscometer LVF, commercially available from Brookfield Engineering Laboratories, Inc, USA.

A representation of the viscosity may be obtained in accordance with Examples 10 and 11. The time for the suspension to cross the 15 cm line is proportional to the viscosity. Preferably, the time at 15 °C is between 5 s and 60 s. This provides a suspension that is able to sufficiently flow, and differentiates from solid gels. More preferably, the time at 15 °C is between 10 and 50 s, even more preferably between 15 and 45 s, even more preferably between 20 and 40 s, still more preferably between 25 and 35 s, most preferably between 28 and 32 s. Each preferably contains between 50 g and 150 g of *Sphagnum* per L of suspension, more preferably between 75 g and 125 g, even more preferably around 100 g. Preferably, the suspension without the *Sphagnum* has a time of between 5 s and 30 s, more preferably between 10 s and 20 s, even mor preferably between 12.5 s and 17.5 s. The thickening agent can be selected to provide the desired propertied. This can provide the desirable properties of the suspension which permit the *Sphagnum* to be sufficiently held and suspended. Preferably, the time at a temperature between 10 °C and 25 °C is between 5 s and 60 s, more preferably between 10 and 50 s.

Optionally, the fluid solution has a pH of between 3.5 and 6.5, preferably between 5.0 and 6.0.

To provide a sustainable way of cultivating *Sphagnum,* it is preferable that the source is not translocated from a wild site. For example, when applying *Sphagnum* to restore a damaged peatland or for production of growing media, it is clearly preferable that the *Sphagnum* used is not translocated from another wild site such as a peatland. Preferably, the *Sphagnum* is sourced from a cultivated source. In one example, the *Sphagnum* could be cultivated or grown in a greenhouse. In some examples, this could originate from a wild site, but the *Sphagnum* can be bulked up by cultivation in a greenhouse, meaning that more *Sphagnum* can be used for a certain amount taken from the wild site.

Optionally, the fragments of *Sphagnum* are cultivated *in vitro.* This means that the *Sphagnum* has been propagated under controlled laboratory conditions, such as in a culture vessel such as a petri dish, a test tube, or other sterile container. Preferably, the *in vitro Sphagnum* has been cultivated using tissue culture techniques. Preferably, the fragments of *Sphagnum* are micropropagated. This means that the *Sphagnum* was grown using clonal tissue culture techniques to produce genetically identical plantlets. This is typically performed in a laboratory by initiating *Sphagnum in vitro.* This can require only a small amount of wild harvested material, reducing the environmental impact. The *in vitro Sphagnum* can then be chopped to form the plurality of fragments of *Sphagnum* for use in the suspension of the present disclosure.

Optionally, the fragments of *Sphagnum* have subsequently been cultivated *in vivo.* As used herein, cultivating *"in vivo"* preferably means cultivating outside of laboratory conditions, such as outside of *in vitro* or tissue culture conditions. For example, after growing *in vitro* such as by micropropagation, the *Sphagnum* may be transferred to and grown *in vivo* such as in a greenhouse. Preferably, the *Sphagnum* is cultivated *in vivo* for at least one month, preferably at least four months. The *Sphagnum* can then be harvested and chopped to form the plurality of fragments of *Sphagnum* for use in the suspension of the present disclosure.

Providing fragments of *Sphagnum may* involve chopping strands of *Sphagnum* to provide the desired length. For example, a desired length may be between 5 mm and 30 mm.

Optionally, at least 50% by mass of the fragments of *Sphagnum* have a length of at least 5 mm. During the chopping process, smaller fragments may form due to inefficiencies in the chopping mechanism and the tendency of branches and leaves to break off due to the fragility of the strands. By suitable choice of a chopping mechanism, this effect can be mitigated, but a small proportion of the fragments will have a length shorter than desired. For example, it is desirable to minimise the number of fragments with a length less than 5 mm. These fragments tend to have fewer potential growing points, are less sturdy, and have a lower probability of quality establishment. Preferably, less than 60% by mass of the fragments of *Sphagnum* in the suspension will have a length less than 5 mm, more preferably less than 50%, still more preferably less than 40%, yet more preferably less than 35%. Measurements of the number of fragments less than 5 mm are shown in Example 3 below.

Optionally, the fragments of *Sphagnum* have a mean length of between 5 mm and 50 mm, preferably between 5 mm and 30 mm. Optionally, fragments of *Sphagnum* having a length of at least 5 mm have a mean length of between 5 mm and 30 mm. Optionally, fragments of *Sphagnum* having a length of at least 5 mm have a mean length of between 5 mm and 50 mm. Preferably, the fragments of *Sphagnum* having a length of at least 5 mm have a mean length of between 5 mm and 25 mm, more preferably between 7.5 mm and 20 mm, even more preferably between 7.5 and 15 mm. Measurements of the lengths of fragments of *Sphagnum* are shown in Example 4 below.

Optionally, the suspension comprises at least 1000 fragments of *Sphagnum* having a length of at least 5 mm per L of fluid solution. Preferably, the suspension comprises at least 2000 fragments of *Sphagnum* having a length of at least 5 mm per L of fluid solution. Measurements of the numbers of fragments of *Sphagnum* are shown in Example 3 below.

Optionally, the suspension comprises a total mass of fragments of *Sphagnum* of at least 50 g per L of fluid solution. Optionally, the suspension comprises a total mass of fragments of *Sphagnum* of between 50 g and 100 g per L of fluid solution, preferably about 100 g per L. Optionally, the suspension comprises a total mass of fragments of *Sphagnum* of at least 25 g per L of fluid solution. Optionally, the suspension comprises a total mass of fragments of *Sphagnum* of less than 500 g per L of fluid solution. Optionally, the suspension comprises a total mass of fragments of *Sphagnum* of less than 400 g per L of fluid solution, preferably less than 300 g per L. The total mass can be calculated by compressing the *Sphagnum* to a standardised mass per volume by compressing with a force of 16 g / cm² to remove excess water to ensure a standardised mass.

Optionally, the suspension comprises a total dry mass of fragments of *Sphagnum* of at least 1 g per L of fluid solution. The dry mass can be calculated by drying the *Sphagnum* to remove the water. As is standard in the art, the drying can be performed by heating the *Sphagnum* at around 110 °C for at least 24 hours. Alternatively, the drying can be performed by drying at around 25 °C in a humidity of less than 50 % until no further weight loss is measured. This ensures that all water has been evaporated, and that remaining is the dry mass of the *Sphagnum.* Of course, this will kill the *Sphagnum,* so this merely provides a useful method for calculation of the dry mass. Measurements of the total dry mass of fragments of *Sphagnum* are shown in Example 6 below.

Optionally, the fragments of *Sphagnum* have a mean stem diameter of between 0.1 mm and 1 mm. Measurements of the stem diameter of fragments of *Sphagnum* are shown in Example 5 below.

According to a second aspect of the present disclosure, there is provided a method of producing a suspension of *Sphagnum,* the method comprising: providing a plurality of fragments of *Sphagnum;* preparing a fluid solution comprising: providing water; and dissolving a thickening agent in the water, wherein the thickening agent comprises a cellulose-based or a starch-based thickening agent; and mixing the plurality of fragments of *Sphagnum* with the fluid solution to suspend the plurality of fragments of *Sphagnum* in the fluid solution.

Disclosed herein is a method of producing a suspension of *Sphagnum,* the method comprising: providing a plurality of fragments of *Sphagnum;* preparing a fluid solution comprising: providing water; and dissolving a thickening agent in the water; and mixing the plurality of fragments of *Sphagnum* with the fluid solution to suspend the plurality of fragments of *Sphagnum* in the fluid solution. Optionally, the suspension further comprises nutrients. Optionally, the nutrients comprise calcium. Optionally, the thickening agent comprises a cellulose-based thickening agent. Optionally, the thickening agent comprises a starch-based thickening agent.

Optionally, the method of the second aspect comprises providing the suspension comprising any of the features described in relation to the suspension of the first aspect.

Features described in relation to the first aspect may be applied to the second aspect alone or in combination, and vice versa. In particular, features of the fragments of *Sphagnum,* the nutrients, and the thickening agent described in relation to the first aspect can be applied to the second aspect, and vice versa.

According to a third aspect of the present disclosure, there is provided a method of providing a seedstock of *Sphagnum* comprising: providing *in vitro Sphagnum;* applying the *Sphagnum* to a growth surface; cultivating the *Sphagnum in vivo* on the growth surface; harvesting the cultivated *Sphagnum* from the growth surface; and chopping the harvested *Sphagnum* to provide seedstock of *Sphagnum* for cultivation, the seedstock comprising a plurality of fragments of the *in vivo Sphagnum.*

Disclosed herein is a method comprising providing *in vitro Sphagnum;* applying the *Sphagnum* to a growth surface; cultivating the *Sphagnum in vivo* on the growth surface; harvesting the cultivated *Sphagnum* from the growth surface; and chopping the harvested *Sphagnum* to provide a plurality of fragments of *in vivo Sphagnum* for cultivation.

The *Sphagnum* may be any one or more of the species of *Sphagnum* disclosed herein. Preferably, the *Sphagnum* is *Sphagnum palustre.*

The *Sphagnum* is *in vitro.* This means that the *Sphagnum* has been propagated under controlled laboratory conditions. Preferably, the *in vitro Sphagnum* has been cultivated using tissue culture techniques. Preferably, the *Sphagnum* has been micropropagated. This means that the *Sphagnum* was grown using clonal tissue culture techniques to produce genetically identical plantlets. Preferably, the *in vitro Sphagnum* has been cultured under sterile conditions or substantially sterile conditions.

The term *"in vitro Sphagnum"* encompasses *Sphagnum* which has been grown under *in vitro* conditions, such as in a laboratory. The *in vitro Sphagnum* is in contrast to *in vivo Sphagnum* which has been grown outside of controlled laboratory conditions, such as in a greenhouse or on a field. Growing *in vitro* isolates the *Sphagnum* and reduces exposure to pathogens, resulting in a cleaner product. Clonal propagation *in vitro* can produce identical plantlets from the original source.

Preferably, the *in vitro Sphagnum* has not been grown *in vivo,* e.g. outside of a laboratory such as in a greenhouse, before being applied to the growth surface. In other words, the term *"in vitro Sphagnum"* preferably does not include *Sphagnum* that has been cultivated outside of laboratory conditions, for example for more than one month, before being applied to the growth surface. However, it will be appreciated that the *in vitro Sphagnum* may be briefly removed from *in vitro* conditions, such as during the preparation of a suspension comprising the *in vitro Sphagnum* as will be described below, for example by removing the *Sphagnum* from the laboratory for chopping into fragments and mixing with a fluid solution to form a suspension of *in vitro Sphagnum,* and that the term *"in vitro Sphagnum"* thus preferably extends to this brief period outside of laboratory conditions.

In this manner, the *in vitro Sphagnum* is clean. The *Sphagnum* can be grown in a nutrient medium and for example supplemented with artificial lighting to culture the *Sphagnum in vitro.* The *Sphagnum* may be grown in culture vessels such as petri dishes which are sealed or substantially sealed to prevent contamination. The exposure of the *in vitro Sphagnum* to contaminants is thus minimal. The cultures and/or equipment may also be sterilised before initiation, reducing the risk of contamination and ensuring a clean product.

Providing micropropagated *in vitro Sphagnum* allows clonal production which provides a sustainable source, with less damage to the environment than harvesting wild *Sphagnum. A* particular desired species can also be selected, for example selecting particular species and/or from a particular location which can be important for restoration and reintroduction purposes. In other cases, particular species that have desirable growth characteristics for *Sphagnum* farming may be selected. For example, *Sphagnum palustre* has been found to be particularly well suited for its physical properties and produces a high productivity harvest and a high volume growing media.

The *Sphagnum* can be applied to a growth surface. The growth surface is a surface suitable for facilitating growth of the *Sphagnum.* For example, the growth surface may comprise a growth substrate such as compost, soil, sand, peat, dried *Sphagnum,* and/or alternative growing media.

The *Sphagnum* can then be cultivated *in vivo* on the growth surface. As used herein, "cultivating" preferably means maintaining the *Sphagnum* in a live state. Cultivating encompasses and preferably refers to facilitating growth of the *Sphagnum.* Preferably, the method comprises growing the *Sphagnum in vivo* on the growth surface. As used herein, cultivating *"in vivo"* preferably means cultivating outside of *in vitro* conditions, or in other words outside of controlled laboratory conditions. For example, *in vivo* cultivating encompasses cultivating in a greenhouse or in a field.

The *Sphagnum* can then be harvested from the growth surface. After a desired period of time cultivating the *Sphagnum,* the *Sphagnum* can be removed from the growth surface. This may be determined by the length of time, or by the size or coverage of the *Sphagnum* after a sufficient amount of growth.

The harvested *Sphagnum* can then be chopped to provide a seedstock of *Sphagnum* for cultivation, the seedstock comprising a plurality of fragments of the *in vivo Sphagnum.* In other words, the harvested *in vivo Sphagnum* is chopped into a plurality of fragments which form the seedstock. The purpose of the chopping is to provide fragments of a desired size for further growth. Preferably, the chopped fragments are still alive and capable of growth. Preferably, the harvested *Sphagnum* is alive when chopped. In particular, the harvested *Sphagnum* is preferably not dried out or dead before chopping.

The plurality of fragments of *in vivo Sphagnum* thus provide the seedstock. The seedstock acts as a supply of *Sphagnum* for further growth. For example, the seedstock can be used by spreading the fragments on a growth surface for growth. In other words, the term "seedstock" does not mean seeds per se, but rather vegetative pieces of *Sphagnum* which can be used for further growth. As *Sphagnum* can be grown from fragments of vegetative material, fragments can be used as a seedstock. The seedstock can also include a suspension of *Sphagnum* as will be described below, which can act as a particularly effective means for spreading the *Sphagnum.*

The resultant *in vivo Sphagnum* in the seedstock is particularly suitable for further growth. Because it originated from an *in vitro* source, it retains the properties of that source. For example, the material is clonal and its propagation is sustainable, and it is still relatively clean given appropriate *in vivo* conditions of cultivation.

Furthermore, the resultant fragments of *in vivo Sphagnum* in the seedstock have significantly different characteristics to the *in vitro Sphagnum.*

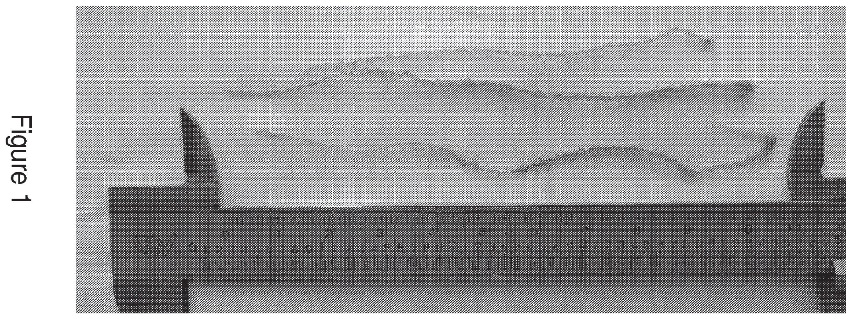

*Sphagnum* typically grows from individual discrete growing points called innovations. In whole plants of wild *Sphagnum,* each strand grows primarily from a large capitulum at the top of the stem. This results in a very small number of growing points in a defined area of *Sphagnum,* largely constrained by the number of capitula. *In vitro Sphagnum* forms a long thin strand with a small capitulum and without any branches. An example strand of *in vitro Sphagnum* is shown in Figure 1. The stem comprises small leaves spaced around 0.5 - 1 mm apart along the length of the stem. If *in vitro Sphagnum* is used as a seedstock, many of the advantages of *in vitro* material are realised e.g. a clean and sustainable product. Furthermore, it has been found that if *in vitro Sphagnum* is chopped into a plurality of fragments for the seedstock, each leaf has the potential to form an innovation. In other words, each leaf is a potential innovation. On each fragment, one or more leaves will typically form an innovation and begin to grow when applied to a growth surface. This results in a large number of innovations compared to only the capitulum growing. Providing a large number of innovations is useful in a seedstock as it increases the initial coverage of *Sphagnum.* However, the inventors have found that the seedstock can be further improved by cultivating the *in vitro Sphagnum in vivo,* and then using the subsequent material as the seedstock.

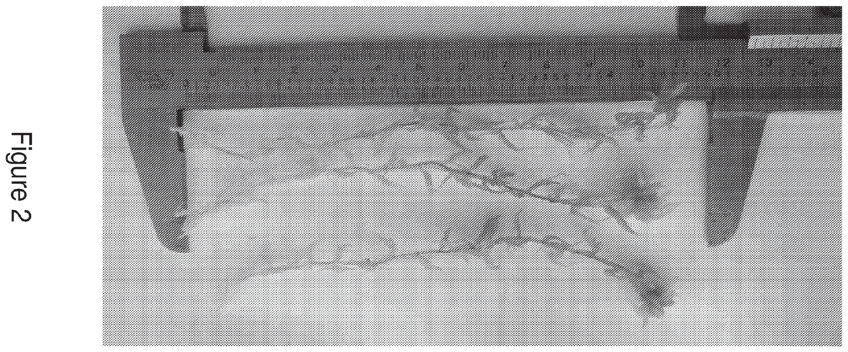

In contrast to *in vitro Sphagnum,* the resultant strands of *in vivo Sphagnum* are physically different. An example strand of *in vivo Sphagnum* is shown in Figure 2. The *in vivo Sphagnum* comprises a number of branches along the length of the stem, more similar to wild *Sphagnum.* The branches are typically arranged around 5 mm apart along the length of the stem. When the *in vivo Sphagnum* is chopped and the seedstock is applied to a growth surface, the branches can form innovations. In other words, the branches are potential innovations, rather than the leaves. By chopping the fragments to generally provide around one branch per fragment, a potential innovation can be provided per fragment. This results in a slightly lower number of total innovations compared to the same amount of *in vitro* material. Therefore, it might be expected that the growth performance would be worse. However, surprisingly, the *in vivo Sphagnum* provides an improvement of growth compared to the *in vitro Sphagnum,* especially over the initial establishment growth phase.

It has been found that the resulting fragments of *in vivo Sphagnum* are more robust than the *in vitro Sphagnum.* In particular, the stem diameter of the fragments is much larger than the diameter of the stems of *in vitro Sphagnum.* This allows for better water and nutrient absorption and transport, which promote better growth when the *in vivo* seedstock is applied to a growth surface. The thicker stems provide a stronger material which is more resilient to drought and contaminants, and has better water holding capacity.

It has also been found that when the *in vivo Sphagnum* in the seedstock is applied to a growth surface, the size of each innovation is also much larger than the size of the innovations of the *in vitro Sphagnum.* As each individual growing point is much larger, this means that it is more hardy and resilient when further cultivated. This makes it particularly well-suited for initial establishment, especially in harsh conditions such as on a peatland. The *in vivo Sphagnum* originating from the *in vitro* source still has a higher density of innovations than wild-harvested *Sphagnum.* The cultivation phase of the present method hence results in the *Sphagnum* becoming more adapted to the environment, so that when the seedstock is applied to a surface and grown on, it establishes faster and more effectively. The larger innovations are less dominated by competitors such as algae, other mosses, or liverworts, and result in improved efficiency of establishment over *in vitro Sphagnum.* The thicker stems provide an improved water holding capacity which can improve establishment in poor conditions. The *in vivo Sphagnum* of the seedstock thus provides an optimum between the number of innovations from *the in vitro* material, and the larger and more robust innovations from cultivating *in vivo.*

The *in vivo* fragments are faster to establish because of their larger growing points. The fragments are also more resistant to the environmental conditions due to their exposure during the cultivating step of the method. This also avoids the effect of shock to the *in vitro Sphagnum* after immediate spreading directly from the laboratory.

Measurements of the comparative growth of fragments of *in vitro Sphagnum* and fragments of *in vivo Sphagnum* are shown in Examples 1 and 2 below.

Optionally, the fragments of *in vivo Sphagnum* have a mean stem diameter which is at least 50% thicker than a mean stem diameter of the *in vitro Sphagnum.* Optionally, the fragments of *in vivo Sphagnum* have a mean stem diameter which is at least 20% thicker than a mean stem diameter of the *in vitro Sphagnum,* preferably at least 30% thicker, more preferably at least 40%, still more preferably at least 50%, even more preferably at least 60%, yet more preferably at least 70%. Optionally, at least 25% of the fragments of *in vivo Sphagnum* have a stem diameter at least 25% thicker than a mean stem diameter of the *in vitro Sphagnum,* preferably at least 50% thicker. Optionally, at least 50% of the fragments of *in vivo Sphagnum* have a stem diameter at least 25% thicker than a mean stem diameter of the *in vitro Sphagnum,* preferably at least 50% thicker.

Optionally, the fragments of *in vivo Sphagnum* have a mean stem diameter of at least 0.4 mm. Optionally, the fragments of *in vivo Sphagnum* have a mean stem diameter of at least 0.3 mm, preferably at least 0.35 mm, more preferably at least 0.4 mm, even more preferably at least 0.45 mm. Optionally, the *in vitro Sphagnum* has a mean stem diameter of less than 0.3 mm. Optionally, the in vitro *Sphagnum* have a mean stem diameter of less than 0.45 mm, preferably less than 0.4 mm, more preferably less than 0.35 mm, even more preferably less than 0.3 mm. Optionally, the mean stem diameter of the fragments of *in vivo Sphagnum* is at least 0.05 mm larger than the mean stem diameter of the *in vitro Sphagnum,* preferably at least 0.075 mm, more preferably at least 0.1 mm, even more preferably at least 0.15 mm, still more preferably at least 0.2 mm.

Measurements of the mean stem diameter are shown in Example 5 below.

Optionally, the fragments of *in vivo Sphagnum* have a mean length of between 5 and 50 mm. Optionally, the fragments of *in vivo Sphagnum* have a mean length of between 5 and 30 mm, preferably between 10 and 20 mm. It is preferable to maximise the number of fragments of *in vivo Sphagnum* with a length of at least 5 mm to maximise the number of fragments with a branch with the potential to form an innovation. Optionally, at least 25% of the fragments of *in vivo Sphagnum* have a length of at least 5 mm, preferably at least 30%, more preferably at least 40%, even more preferably at least 50%. Optionally, at least 25% of the fragments of *in vivo Sphagnum* have a length between 5 mm and 50 mm, preferably at least 30%, more preferably at least 40%, even more preferably at least 50%.

Optionally, the providing *in vitro Sphagnum* comprises chopping *in vitro Sphagnum* into a plurality of fragments of *in vitro Sphagnum.* The *in vitro Sphagnum* fragments may have any length described in relation to the *in vivo Sphagnum.* For example, the fragments of *in vitro Sphagnum* may have a mean length of between 5 and 50 mm, preferably between 5 and 30 mm.

Optionally, the providing *in vitro Sphagnum* comprises mixing the plurality of fragments of *in vitro Sphagnum* with a first fluid solution to provide a first suspension of *in vitro Sphagnum.*

For example, the fragments of *in vitro Sphagnum* may be mixed with the first fluid solution by stirring with a mechanical stirrer, or by stirring with a utensil operated by hand.

Optionally, the first fluid solution comprises water and a thickening agent dissolved in the water. The suspension may comprise any of the features of the suspension described herein, such as in the first aspect of the present disclosure. For example, the first fluid solution may comprise any of the features described in relation to the fluid solution of the first aspect of the present disclosure. For example, the thickening agent may comprise a cellulose-based or a starch-based thickening agent. Preferably, the thickening agent is cellulose-based, more preferably is hydroxyethyl cellulose, such as in a concentration described above. For example, the fluid solution may comprise nutrients as described above.

Optionally, the suspension of *in vitro Sphagnum* comprises at least 50 g of *in vitro Sphagnum* per L of the first fluid solution. Optionally, between 50 g and 150 g, preferably around 100 g. Optionally, the first fluid solution comprises a total dry mass of fragments of *Sphagnum* of at least 1 g per L of water, more preferably at least 1.5 g, even more preferably at least 2 g.

Optionally, the applying comprises applying the suspension of *in vitro Sphagnum* onto the growth surface at a density of between 0.5 and 5 L / m². Preferably, the applying comprises applying the suspension of *in vitro Sphagnum* onto the growth surface at a density of between 0.5 and 3 L / m². More preferably, the applying comprises applying the suspension of *in vitro Sphagnum* onto the growth surface at a density of between 1 and 3 L / m². Even more preferably, the suspension is applied onto the growth surface between 1.5 and 2.5 L / m², most preferably approximately 2 L / m².

Optionally, the applying comprises spraying the suspension of *in vitro Sphagnum* onto the growth surface.

Optionally, the spraying comprises pumping the suspension of *in vitro Sphagnum* through an aperture to hit a deflecting plate. Preferably, the aperture is less than 10 mm in diameter, more preferably between 5 mm and 10 mm, most preferably about 6 mm. The deflecting plate provides spreading over a greater area and a more even spread. For example, the suspension may be sprayed by using a peristaltic pump. A peristaltic pump evenly pumps the suspension without the fragments of *Sphagnum* tangling in the pump. For fragments sizes below about 30 mm, a peristaltic pump has been found to provide optimum pumping without tangling. Alternatively, the pump may be a vane pump having smooth soft vanes to prevent damage to the *Sphagnum.*

Optionally, the applying comprises applying the suspension in discrete amounts onto the growth surface. For example, the suspension may be pumped onto individual cells in a plug cell tray. This results in small clumps of *Sphagnum* growing on individual plugs of the growth surface, such as a growing medium.

Optionally, the growth surface comprises compost, soil, sand, peat, and/or dried *Sphagnum.* Alternatively, the growth surface may comprise an alternative growing media, such as a peat-free growing media. Preferably, the growth surface comprises peat and/or dried *Sphagnum.* The growth surface is preferably the uppermost surface, for example exposed to the air (before *Sphagnum* is applied). For example, the growth substrate may be a growing medium such as compost, peat, dried *Sphagnum,* and/or alternative growing media. The growth surface may be an outdoor area such as a field or a peatland. Alternatively, the growth surface may be a horticultural growing bed, such as an indoor or outdoor bed. The growth surface may be arranged in a tray, such as a horticultural tray for use in a greenhouse or polytunnel or outdoors. For example, the growth surface may comprise a horticultural growing medium (e.g. a peat-based growing medium) applied to a tray. The tray may be a plug cell tray having individual cells for plugs, or it may be a carpet tray having a single larger region for growing media.

Optionally, the cultivating is carried out under controlled environmental conditions. Optionally, the controlled environmental conditions controlled comprise at least one of: irrigation, nutrient supply, lighting, shading, temperature, and/or humidity. For example, the cultivating may be carried out in an indoor growing room such as a greenhouse or a polytunnel. Optionally, the indoor growing room is heated. Optionally, the indoor growing room comprises artificial lighting to replace or supplement natural lighting for growth of *Sphagnum.* In one example, the cultivating is carried out in a greenhouse, optionally wherein the greenhouse is temperature and/or humidity controlled.

Optionally, the cultivating comprises irrigating the *Sphagnum.* Optionally, the irrigating comprises applying water to the *Sphagnum* at a rate of at least 1 L / m² / day. Optionally, the irrigating is performed using spray irrigation such as using small droplet sprinklers or a misting system. The amount of irrigation can be dependent on water loss by evaporation. In other words, humidity airflow can affect the water application required, and more or less water can be applied as necessary.

Optionally, the cultivating comprises applying nutrients to the *Sphagnum.* Optionally, the nutrients are contained in water supplied via irrigation. For example, the nutrients may be the same or different to nutrients provided in the suspension. Optionally, the nutrients are applied after cultivating for at least two weeks. For example, as the suspension contains a supply of nutrients, in some cases it is not necessary to supply nutrients for an initial period of cultivation. The nutrients may comprise any of the nutrients in any of the concentrations or ranges as described in relation to the first or second aspect of the present disclosure.

Optionally, the cultivating is carried out for at least one month. Preferably, the cultivating is carried out for at least four months. More preferably, the cultivating is carried out for between four and six months. In some cases, the cultivating can be carried out for longer, such as when outside and the *Sphagnum* growth is slower.

Optionally, the chopping the harvested *Sphagnum* comprises chopping such that at least 50 % by weight of the fragments of *in vivo Sphagnum* have a length of at least 5 mm. Optionally, at least 30 %, preferably at least 40 %, more preferably at least 50 % even more preferably at least 60 %.

Optionally, the providing the seedstock of *Sphagnum* comprises mixing the plurality of fragments of *in vivo Sphagnum* with a second fluid solution to provide a suspension of *in vivo Sphagnum.* For example, the second fluid solution may be the same as the first fluid solution. In other cases, the second fluid solution may be different. For example, it may have a different thickening agent or concentration, or a different nutrient composition. Although referred to as the "second" fluid solution, in some examples the seedstock may comprise a fluid solution, while the *in vitro Sphagnum* is not suspended in a fluid solution. In other words, the second fluid solution is not limited to the inclusion of the first fluid solution.

Optionally, the second fluid solution comprises water and a thickening agent dissolved in the water. For example, the second fluid solution may comprise any of the features of the fluid solution disclosed herein, such as in the first aspect of the present disclosure. For example, the thickening agent may comprise a cellulose-based or a starch-based thickening agent. For example, the second fluid solution may comprise nutrients such as calcium. Preferably, the second fluid solution comprises hydroxyethyl cellulose as a thickening agent. Concentrations of hydroxyethyl cellulose described herein may be used. Preferably, the second fluid solution comprises between 3 g and 12 g of hydroxyethyl cellulose per L of water, more preferably between 4 g and 11 g, even more preferably between 5 g and 10 g.

In some examples, the second fluid solution comprises more thickening agent than the first fluid solution. In one example, the second fluid solution comprises around 0.5 g more of hydroxyethyl cellulose per L of water. Optionally, the second fluid solution comprises a total dry mass of fragments of *Sphagnum* of at least 1 g per L of water.

In other words, the seedstock comprises the suspension of *Sphagnum.* As described herein, the suspension provides a particularly effective means for carrying the *Sphagnum* and for spreading it.

Optionally, the method further comprises applying the seedstock of *Sphagnum* to a growth surface.

According to a fourth aspect of the present disclosure, there is provided a method comprising: performing the method of the third aspect, and applying the seedstock of *Sphagnum* to a growth surface. For completeness, the method comprises: providing *in vitro Sphagnum;* applying the *Sphagnum* to a growth surface; cultivating the *Sphagnum in vivo* on the growth surface; harvesting the cultivated *Sphagnum* from the growth surface; chopping the harvested *Sphagnum* to provide seedstock of *Sphagnum* for cultivation, the seedstock comprising a plurality of fragments of the *in vivo Sphagnum;* and applying the seedstock of *Sphagnum* to a growth surface. The growth surface may comprise any of the features described above in relation to the growth surface on which the first suspension is applied. For example, applying the seedstock of *Sphagnum* to a growth surface may comprise spraying the suspension of *in vivo Sphagnum* on the growth surface. Applying the seedstock to a growth surface allows cultivation of the *Sphagnum* in the seedstock.

Optionally, the method further comprises cultivating the *Sphagnum* in the seedstock *in vivo* on the growth surface. The cultivating may comprise any of the features described above in relation to cultivating the *in vitro Sphagnum in vivo* on the growth surface. For example, the cultivating may be performed indoors, such as in a greenhouse which may be heated.

It has been found that performing this further second cultivation step results in *Sphagnum* that is even more improved. In particular, the *Sphagnum* is more resilient after growing longer *in vivo.* This cultivation results in second generation *in vivo Sphagnum* which is distinguished from first generation *in vivo Sphagnum* which results from the first cultivation step. This second generation material can then be used for growth.

Optionally, the method further comprises harvesting the *Sphagnum* of the seedstock from the growth surface. This *Sphagnum* can then be planted at a site, for example for peatland restoration. For example, clumps of *Sphagnum* can be taken after at least four months growth and planted at a site, which can provide faster establishment than covering an area with chopped fragments. This second generation *Sphagnum* will have desirable properties in that the establishment is improved compared to *in vitro* material and wild harvested *Sphagnum,* and also further improved compared to first generation *in vivo* material.

Optionally, the method comprises providing a growing medium comprising the harvested *Sphagnum* of the seedstock, comprising drying the harvested *Sphagnum* of the seedstock.

Optionally, the method further comprises chopping the harvested *Sphagnum* from the seedstock to provide a second seedstock of *Sphagnum* for cultivation, the second seedstock comprising a plurality of fragments of second generation *in vivo Sphagnum.* This provides a further seedstock containing second generation *in vivo Sphagnum,* which provides a useful mechanism to encourage rapid establishment from small fragments. For example, the *Sphagnum* may be chopped as described herein and to lengths described herein.

Optionally, the providing the second seedstock comprises mixing the plurality of fragments of second generation *in vivo Sphagnum* with a third fluid solution to provide a suspension of second generation *in vivo Sphagnum.* The third fluid solution may comprise any of the features described above in relation to the first or second fluid solutions, or the fluid solution of the first aspect of the present disclosure.

Optionally, the third fluid solution comprises water and a thickening agent dissolved in the water. Optionally, the thickening agent is a cellulose-based or starch-based thickening agent. Optionally, the thickening agent comprises hydroxyethyl cellulose. Optionally, the third fluid solution comprises between 5 g and 10 g of hydroxyethyl cellulose per L of water. Optionally, the third fluid solution comprises a total dry mass of fragments of *Sphagnum* of at least 1 g per L of water.

This third suspension can then be cultivated such as for producing growing media as described above.

According to a fifth aspect of the present disclosure, there is provided *Sphagnum* obtainable by the method disclosed herein. As will be appreciated, the physical difference between the *in vitro Sphagnum* and the resulting *in vivo Sphagnum* of the seedstock represents a significant and detectable technical alteration imparted by the process. For example, clear differences are shown in Figures 1 and 2.

According to a sixth aspect of the present disclosure, there is provided a seedstock of *Sphagnum* obtainable by the method disclosed herein. The seedstock is detectably different compared to prior *Sphagnum* as the second generation *Sphagnum* has visible differences which lead to faster establishment and growth rate. Moreover, the seedstock is different as it contains a plurality of fragments of such *Sphagnum.*

Disclosed herein is a seedstock of *Sphagnum,* comprising a plurality of fragments of *Sphagnum;* wherein the *Sphagnum* is *in vitro Sphagnum* which has been applied to a growth surface and cultivated *in vivo* on the growth surface, and subsequently chopped to provide the plurality of fragments of *Sphagnum.*

According to a seventh aspect of the present disclosure, there is provided a seedstock of *Sphagnum,* comprising: a plurality of fragments of *Sphagnum;* wherein at least 25 % of the fragments of *Sphagnum* have a length of between 5 mm and 50 mm; and wherein at least 25 % of the fragments of *Sphagnum* have a mean stem diameter of at least 0.4 mm.

This provides an optimum size of fragments as they are large enough to contain a potential growing point while avoiding being too long to tangle while being applied.

Optionally, at least 30 % of the fragments of *Sphagnum* have a length of between 5 mm and 50 mm, preferably at least 40 %, more preferably at least 50 %, even more preferably at least 60 %, still more preferably at least 70 %.

Optionally, at least 30 % of the fragments of *Sphagnum* have a mean stem diameter of at least 0.4 mm, preferably at least 40 %, more preferably at least 50 %. Even more preferably, the fragments of *Sphagnum* have a mean stem diameter of at least 0.4 mm (i.e. all of the fragments).

Optionally, the seedstock comprises a fluid solution, optionally comprising water, and the fragments of *Sphagnum* are suspended in the fluid solution. Optionally, the fluid solution contains a thickening agent as disclosed herein.

Optionally, the seedstock comprises at least 50 g of fragments of *Sphagnum* per L of the fluid solution, preferably at least 75 g, more preferably at least 100 g. This provides a suitable density of growing points. Optionally, the seedstock comprises a total dry mass of fragments of *Sphagnum* of at least 1 g per L of fluid solution, more preferably at least 1.5 g, even more preferably at least 2 g.

Optionally, at least 25 % of the fragments of *Sphagnum* have a stem diameter of at least 0.3 mm, preferably at least 0.35 mm. Preferably, at least 40 % of the fragments of *Sphagnum* have a stem diameter of at least 0.3 mm, more preferably at least 0.35 mm. More preferably, at least 50 % of the fragments of *Sphagnum* have a stem diameter of at least 0.3 mm, more preferably at least 0.35 mm. Even more preferably, a mean diameter of the fragments of *Sphagnum* is at least 0.3 mm, still more preferably at least 0.35 mm.

Optionally, the *Sphagnum* is from an *in vitro* source. Preferably, the *Sphagnum* is micropropagated.

Aspects of the invention may be provided in conjunction with each other and features of one aspect may be applied to other aspects. Any feature in one aspect of the invention may be applied to other aspects of the invention, in any appropriate combination. In particular, specific nutrients, species, and thickening agents may be applied to the various examples and aspects disclosed, and a disclosure of a particular example (e.g. of a particular thickening agent or concentration) in one aspect may be applied to other aspects. It should also be appreciated that particular combinations of the various features described and defined in any aspects of the invention can be implemented and/or supplied and/or used independently. Embodiments related to the method may be applied to the *Sphagnum* obtainable by the method, and *vice versa.*

Other definitions of terms may appear throughout the specification. Before the exemplary embodiments are described in more detail, it is to be understood that this disclosure is not limited to particular embodiments described, and as such may vary. It is also to be understood that the terminology used herein is for the purpose of describing particular embodiments only, and is not intended to be limiting, since the scope of the present disclosure will be defined only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

Embodiments of the disclosure are described below, by way of example only, with reference to the accompanying Figures and Examples.
**Figure 1** shows a photograph of a strand of *in vitro Sphagnum.*
**Figure 2** shows a photograph of a strand of *in vivo Sphagnum.*
**Figure** 3 shows a table of the number of innovations counted when *Sphagnum* was applied to a surface in Example 1.
**Figure** 4 shows a photograph of coverage of *Sphagnum* for Trial 1 of Example 1.
**Figure 5** shows a photograph of coverage of *Sphagnum* for Trial 2 of Example 1.
**Figure 6** shows a photograph of coverage of *Sphagnum* for Trial 3 of Example 1.
**Figure 7** shows a table of the number of innovations counted when *Sphagnum* was applied to a surface outdoors in Example 2.
**Figure** 8 shows a graph of the percentage cover of the *Sphagnum* in Example 2.
**Figure 9** shows a photograph of coverage of *Sphagnum* for Trial 1 of Example 2.
**Figure 10** shows a photograph of coverage of *Sphagnum* for Trial 2 of Example 2.
**Figure 11** shows a photograph of coverage of *Sphagnum* for Trial 3 of Example 2.
**Figure 12** shows a table of the proportion of fragments of *Sphagnum* having a length of at least 5 mm for Trial 1 of Example 3.
**Figure 13** shows a table of the proportion of fragments of *Sphagnum* having a length of at least 5 mm for Trial 2 of Example 3.
**Figure 14** shows a table of the proportion of fragments of *Sphagnum* having a length of at least 5 mm for Trial 3 of Example 3.
**Figure 15** shows a table of the lengths of fragments of *Sphagnum* having a length of at least 5 mm for Trial 1 of Example 4.
**Figure 16** shows a table of the lengths of fragments of *Sphagnum* having a length of at least 5 mm for Trial 2 of Example 4.
**Figure 17** shows a table of the lengths of fragments of *Sphagnum* having a length of at least 5 mm for Trial 3 of Example 4.
**Figure 18** shows a table of the stem diameters of *Sphagnum* for Trial 1 of Example 5.
**Figure 19** shows a table of the stem diameters of *Sphagnum* for Trial 2 of Example 5.
**Figure 20** shows a table of the stem diameters of *Sphagnum* for Trial 3 of Example 5.
**Figure 21** shows a table of the wet weight, dry weight, and water content of fragments of *Sphagnum* for Trial 1 of Example 6.
**Figure 22** shows a table of the wet weight, dry weight, and water content of fragments of *Sphagnum* for Trial 2 of Example 6.
**Figure 23** shows a table of the wet weight, dry weight, and water content of fragments of *Sphagnum* for Trial 3 of Example 6.
**Figure 24** shows a table of the distance between branches for Trial 1 of Example 7.
**Figure 25** shows a table of the distance between branches for Trial 2 of Example 7.
**Figure 26** shows a table of the distance between branches for Trial 3 of Example 7.
**Figure 27** shows a photograph of establishment of Trial 1 of Example 8.
**Figure 28** shows a photograph of establishment of Trial 2 of Example 8.
**Figure 29** shows a graph of growth rates of *Sphagnum* of Trials 1 and 2 of Example 9.
**Figure 30** shows a table of the time for suspensions to flow of Example 10.
**Figure 31** shows a table of the time for suspensions to flow of Example 11.

### EXAMPLES

### EXAMPLE 1

### Suspensions of Sphagnum

### Materials and Methods

Suspensions of *Sphagnum* were prepared in accordance with the present disclosure. Three trials were performed. Trial 1 was *in vitro Sphagnum* taken immediately from the laboratory after 3 months of growth. Trial 2 was first generation *in vivo Sphagnum* harvested after 6 months of growth in a heated indoor greenhouse, where *in vitro Sphagnum* was applied to produce the *in vivo Sphagnum.* Trial 3 was also first generation *in vivo Sphagnum* harvested after 6 months of growth in a heated indoor greenhouse, but the *Sphagnum* was unchopped.

The strands of *Sphagnum* of Trial 1 and 2 were chopped into typical lengths of around 5 to 30 mm by a machine. In Trial 3, the strands were not chopped, and had a typical length of around 50-100 mm.

A fluid solution was then prepared by preparing a nutrient stock solution. Hortimix 15-5-15 was used, commercially available from Hortifeeds, UK. The nutrients were then diluted in water to provide 0.75 g of Hortimix 15-5-15 per L of water.

A thickening agent was then dissolved in the nutrient solution. The thickening agent was hydroxyethyl cellulose, in the form of Natrosol^{™} HX, commercially available from Ashland, USA. 8 mg of hydroxyethyl cellulose was used per L of solution.

The fragments of *Sphagnum* of each trial were then mixed with the solution. This suspended the fragments of *Sphagnum* within the thickened solution. The suspension was then sprayed onto a surface at an application rate of 2 L per m² and cultivated in an indoor heated greenhouse for 4 weeks. Irrigation was supplied by applying water to keep damp, and no further nutrients were applied.

A photograph was taken of each of the three trials. A standardising ring was placed on the *Sphagnum* surface in the photograph for calibrating size. By measuring the size of the ring in the photograph, an adjustment ratio was calculated for calibration. The width and length of the photograph was then measured for each trial, and these values were multiplied together to provide a measured area for each photograph. This area was then multiplied by the adjustment ratio to provide the true area in the photograph.

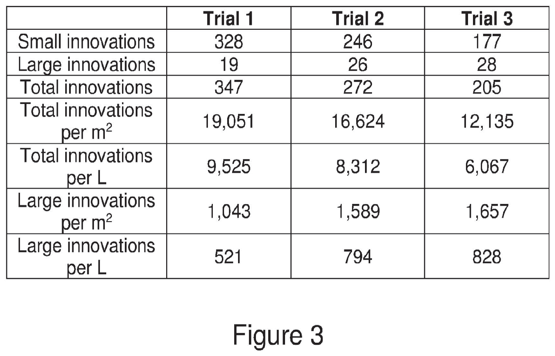

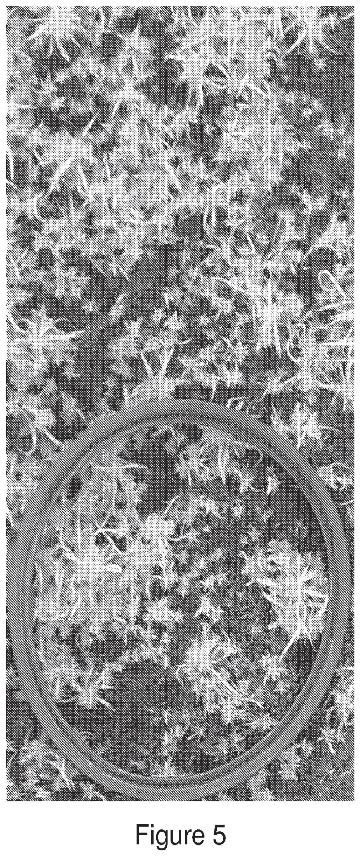

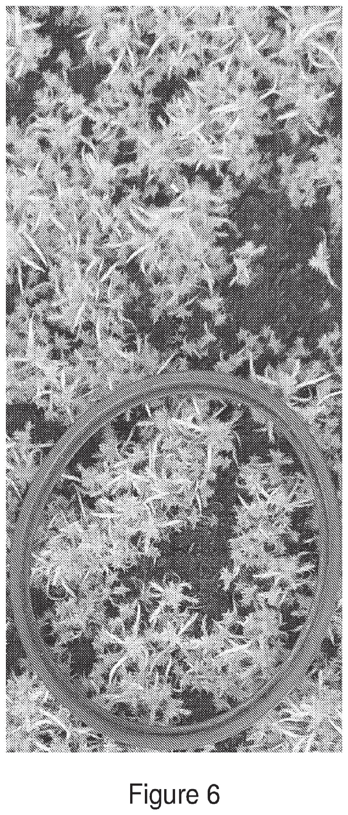

The number of innovations were then counted in each trial. In particular, the number of small innovations and the number of large innovations were counted individually to provide a total number of innovations. Small innovations were defined as those lacking significant branches. By dividing the number of innovations by the area, the total number of innovations per m² was calculated. By dividing the number of innovations per m² by 2 L (the rate of application), the number of innovations per L of suspension was calculated. The results are shown below in Figures 3 to 6.

### Results

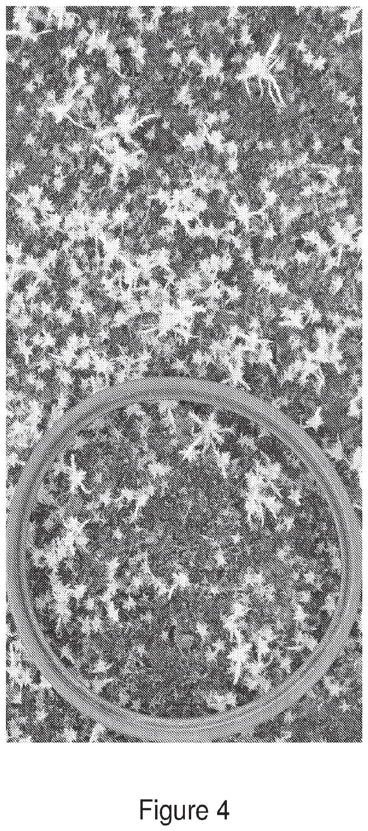

The numerical results are shown in a table in Figure 3. Figures 4 to 6 show the photographs of the innovation results of Trials 1 to 3, respectively. In Trial 1, the total number of innovations per L of suspension for the *in vitro Sphagnum* was 9,525. In Trial 1, the number of large innovations per L of suspension for the *in vitro Sphagnum* was 521.

In Trial 2, the total number of innovations per L of suspension for the *in vivo Sphagnum* was 8,312. In Trial 2, the number of large innovations per L of suspension for the *in vivo Sphagnum* was 794.

In Trial 3, the total number of innovations per L of suspension for the unchopped *in vivo Sphagnum* was 6,067. In Trial 3, the number of large innovations per L of suspension for the unchopped *in vivo Sphagnum* was 828.

The unchopped *in vivo Sphagnum* shows a significant drop in the number of innovations compared to the chopped *in vivo Sphagnum,* showing that the capitula inhibits the number of growing points along the stem. By chopping the *Sphagnum* into appropriate sizes, the number of innovations can be maximised.

Although the number of innovations of *in vitro Sphagnum* is larger than *in vivo Sphagnum,* the number of large innovations is much more in the *in vivo* trial. This shows that the *in vivo Sphagnum* becomes more robust and produces larger innovations which helps initial establishment. Large innovations are particularly important for initial survival in poor conditions, such as on peatlands.

When considering the total establishment in terms of the number of innovations and the size of those innovations, the *in vivo Sphagnum* of Trial 2 shows a significant improvement over the *in vitro Sphagnum* of Trial 1 and the unchopped *Sphagnum* of Trial 3.

### EXAMPLE 2

### Outdoor growth - Suspensions of Sphagnum

### Materials and Methods

Suspensions of *Sphagnum* were prepared in accordance with the present disclosure. Three trials were performed, with three replicate samples for each trial. Trial 1 was *in vitro Sphagnum* taken immediately from the laboratory. Trial 2 was first generation *in vivo Sphagnum* harvested after approximately 6 months of growth in a greenhouse, where *in vitro Sphagnum* was applied to produce the *in vivo Sphagnum.* Trial 3 was second generation *in vivo Sphagnum,* which was first generation *in vivo Sphagnum* subsequently chopped and grown again in the greenhouse for a further 6 months.

The strands of *Sphagnum* of each trial were chopped into typical lengths of around 5 to 30 mm by a machine. A fluid solution was then prepared by preparing a nutrient stock solution. Hortimix 15-5-15 was used, commercially available from Hortifeeds, UK. The nutrients were then diluted in water to provide 0.75 g of Hortimix 15-5-15 per L of water. A thickening agent was then dissolved in the nutrient solution. The thickening agent was hydroxyethyl cellulose, in the form of Natrosol^{™} HX, commercially available from Ashland, USA. 8 mg of hydroxyethyl cellulose was used per L of solution. The fragments of *Sphagnum* of each trial were then mixed with the solution. This suspended the fragments of *Sphagnum* within the thickened solution.

The suspension was then sprayed onto a growth surface in the form of a tray holding a growing medium, at an application rate of 3 L of suspension per m². The tray had a surface area of 2016 cm². The *Sphagnum* was then cultivated outside for 5 weeks. Irrigation was supplied by applying water to keep the *Sphagnum* damp, and no further nutrients were applied.

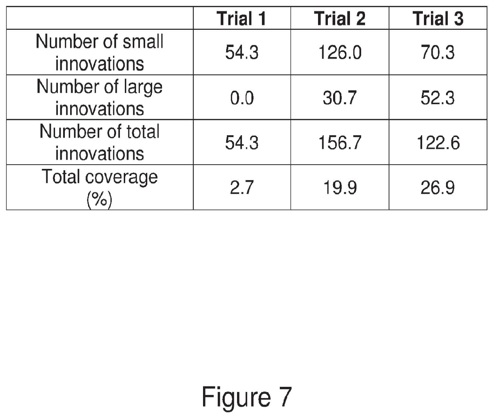

After the 5 weeks of growth outdoors, a photograph was taken of each of the three trials. The number of innovations per quarter tray were then counted in each trial. In particular, the number of small innovations and the number of large innovations were counted individually to provide a total number of innovations. Large innovations were generally where capitula had begun to grow well. Small innovations were defined as those lacking significant branches. Small innovations had a typical size of 0.25 cm², whereas large innovations had a typical size of 2.25 cm². The area of the small innovations and large innovations for each trial was then calculated by multiplying the number by the average size. The area of the innovations was then summed and divided by the known area of the tray, providing the percentage coverage of innovations over the area. The results are shown below in Figures 7 to 11.

### Results

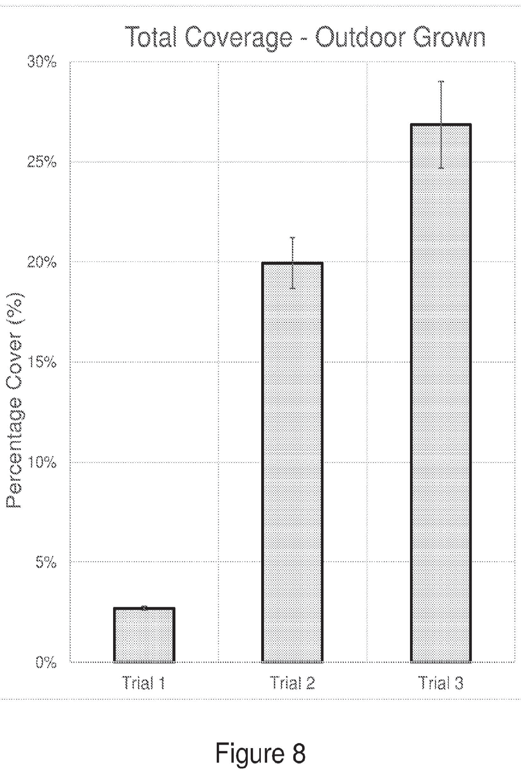

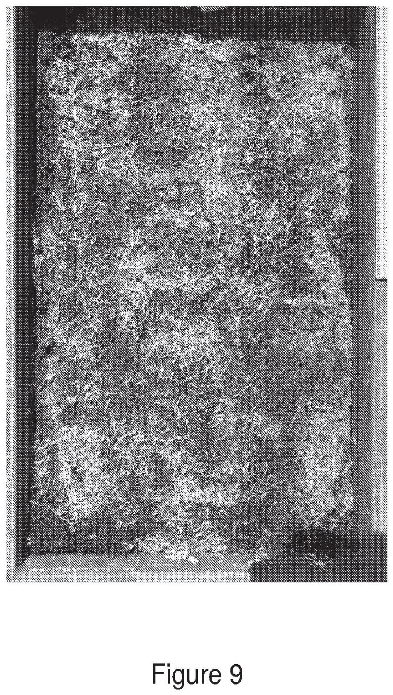

The numerical results are shown in a table in Figure 7. The percentage coverage results are shown in a graph in Figure 8. Figures 9 to 11 show the photographs of the innovation results of a replicate of Trials 1 to 3, respectively. In Trial 1, the number of small innovations per quarter tray (504 cm²) for the *in vitro Sphagnum* was 54.3 with an error, based on the variation in the replicate trials, of 2.0. The number of large innovations per quarter tray for the *in vitro Sphagnum* was 0.0 with an error of 0.0. The percentage coverage of small and large innovations was 2.7 % with an error of 0.1 %.

In Trial 2, the number of small innovations per quarter tray for the *in vivo Sphagnum was* 126.0 with an error of 20.1. In Trial 2, the number of large innovations per quarter tray was 30.7 with an error of 1.8. The percentage coverage of small and large innovations was 19.9 % with an error of 1.3 %.

In Trial 3, the number of small innovations per quarter tray for the second generation *in vivo Sphagnum* was 70.3 with an error of 5.4. In Trial 3, the number of large innovations per quarter tray was 52.3 with an error of 4.8. The percentage coverage of small and large innovations was 26.9 % with an error of 2.2 %.

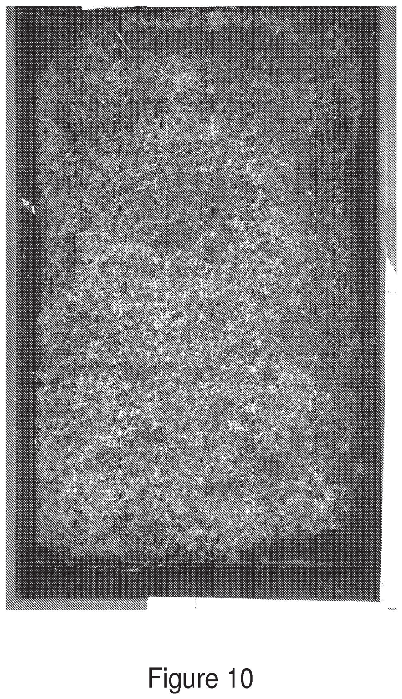

The percentage coverage from innovations increases significantly between the *in vitro Sphagnum* of Trial 1 and the *in vivo Sphagnum* of Trial 2 that had undergone the method of the present disclosure. As shown in Figure 10, not only is the total coverage larger, but there are more larger innovations (capitula) than Figure 9. This reduces the time for establishment and further improves the survival rate. This confirms that, especially in poor conditions such as outdoor establishment, the *in vivo Sphagnum* achieves better initial establishment and obtains a higher coverage. This is particularly important for applying *Sphagnum* for restoration of peatlands, as typically *Sphagnum* will have to be applied to a harsh environment, so survival in the absence of shelter, heat, and nutrients, for example, is hugely beneficial.

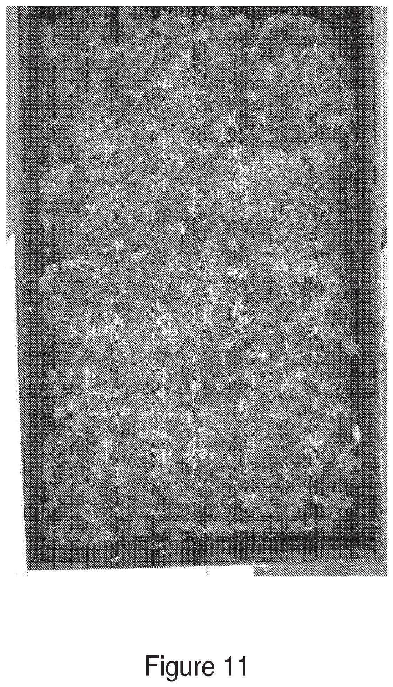

The coverage and number of large innovations is further increased with Trial 3, as shown in Figure 11. The further indoor cultivation and chopping steps imparted to the second generation *Sphagnum* further toughens the *Sphagnum* to make it more resilient, leading to better establishment.

### EXAMPLE 3

### Number of fragments of Sphagnum

### Materials and Methods

Trial 1 was *in vitro Sphagnum* taken immediately from the laboratory after 4 months of growth. Trial 2 was first generation *in vivo Sphagnum* harvested after 6 months of growth in a heated indoor greenhouse, where *in vitro Sphagnum* was applied to produce the *in vivo Sphagnum.* Trial 3 was second generation *in vivo Sphagnum* harvested after 6 months of growth in a heated indoor greenhouse, where the first generation *in vivo Sphagnum* was harvested after 6 months of growth in a heated indoor greenhouse and applied to produce the second generation *in vivo Sphagnum.* Samples of 1 g of *Sphagnum* were harvested. Water content was standardised by compressing the *Sphagnum* with a force corresponding to 16 g / cm² to provide a standardised mass per volume. Three samples were taken for Trial 1, and five samples were taken for Trial 2 and 3.

The samples were floated in water and all fragments having a length of at least 5 mm were selected for counting. The remaining material was strained in a sieve and removed with tweezers for weighing.

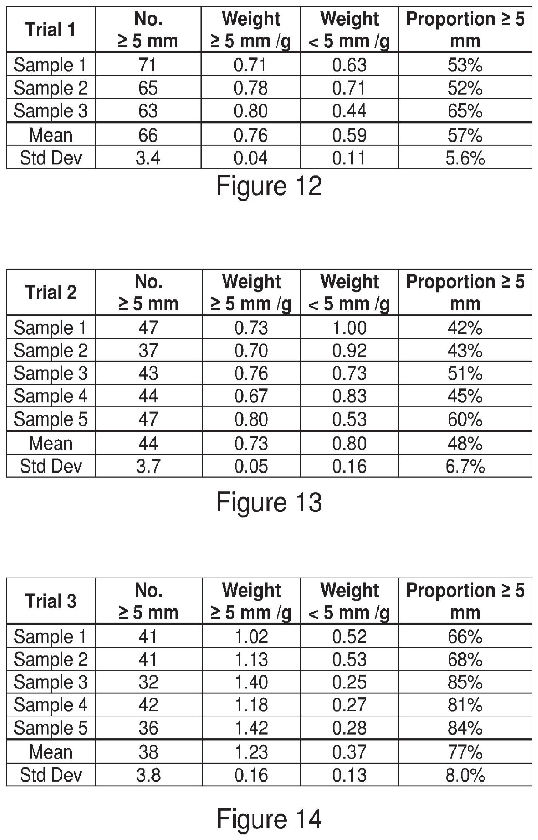

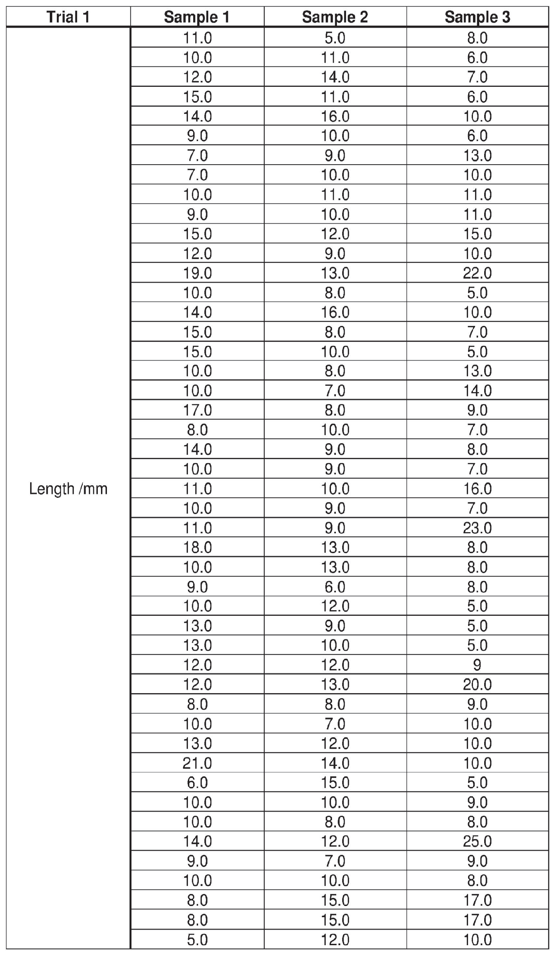

The weight of the fragments having a length of at least 5 mm was measured, and the number of fragments was counted. The weight of the fragments of less than 5 mm was also measured. The weight was standardised by draining until no more water drained by gravity, and then weighing. The proportion of fragments having a length of at least 5 mm by weight for each sample was then calculated. This process was repeated for each sample in each trial. The mean and standard deviations for each trial were then calculated. The number of fragments having a length of at least 5 mm per L of suspension was then calculated for each trial by multiplying the average number of fragments counted per sample and multiplying by 100 to provide the number of fragments per 100 g (approximately per L of suspension). The results are shown in Figures 12 to 14.

### Results

The numerical results are shown in tables in Figures 12 to 14 for Trials 1 to 3, respectively. In Trial 1, the mean proportion of fragments of at least 5 mm by weight for the *in vitro Sphagnum* was 57%. In Trial 1, the mean number of fragments of at least 5 mm per L of suspension for the *in vitro Sphagnum* was 6,633.

In Trial 2, the mean proportion of fragments of at least 5 mm by weight for the first generation *in vivo Sphagnum* was 48%. In Trial 2, the mean number of fragments of at least 5 mm per L of suspension for the first generation *in vivo Sphagnum* was 4,360.

In Trial 3, the mean proportion of fragments of at least 5 mm by weight for the second generation *in vivo Sphagnum* was 77%. In Trial 3, the mean number of fragments of at least 5 mm per L of suspension for the second generation *in vivo Sphagnum* was 3,840.

### EXAMPLE 4

### Lengths of fragments of Sphagnum

### Materials and Methods

Trial 1 was *in vitro Sphagnum* taken immediately from the laboratory after 4 months of growth. Trial 2 was first generation *in vivo Sphagnum* harvested after 6 months of growth in a heated indoor greenhouse, where *in vitro Sphagnum* was applied to produce the *in vivo Sphagnum.* Trial 3 was second generation *in vivo Sphagnum* harvested after 6 months of growth in a heated indoor greenhouse, where the first generation *in vivo Sphagnum* was harvested after 6 months of growth in a heated indoor greenhouse and applied to produce the second generation *in vivo Sphagnum.* Samples of approximately 1 g of *Sphagnum* were harvested. Water content was standardised by compressing the *Sphagnum* with a force corresponding to 16 g / cm² to provide a standardised mass per volume. Three samples were taken for each of the trials.

The samples were floated in water and all fragments having a length of at least 5 mm were selected for counting.

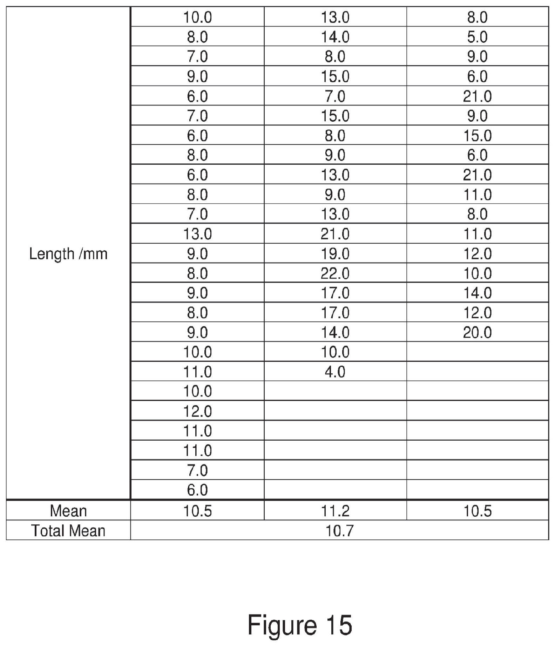

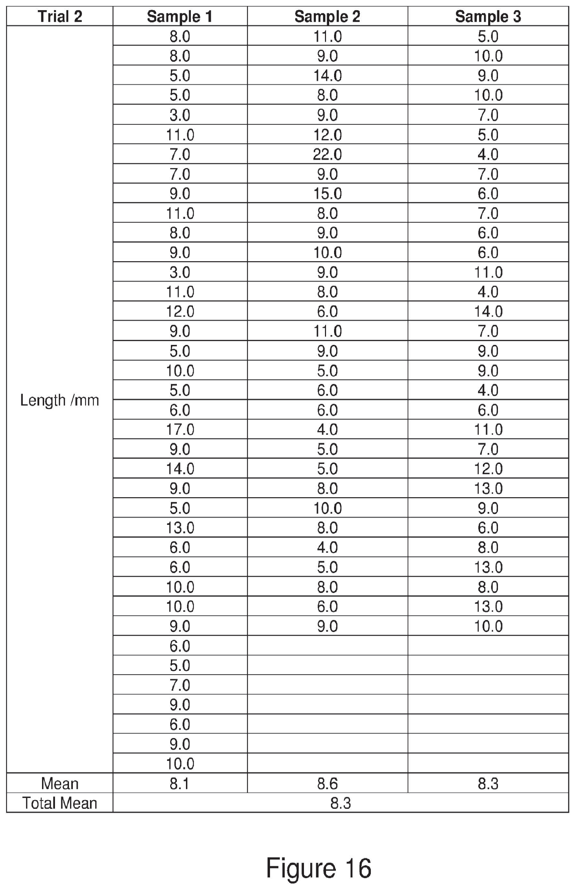

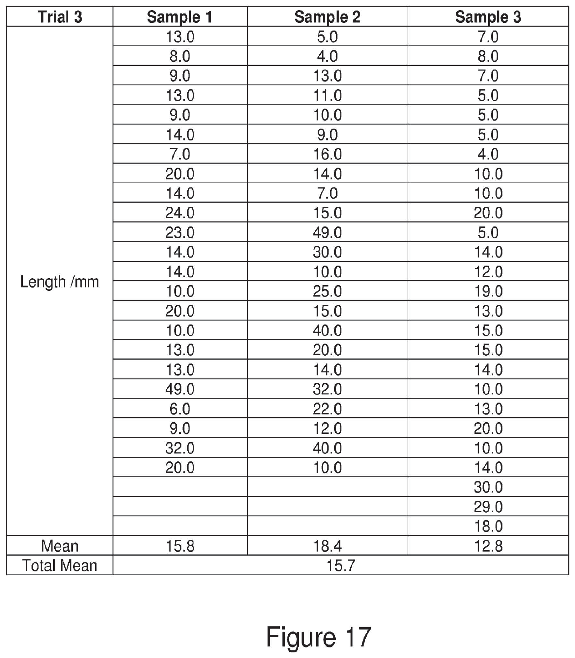

The lengths of the fragments of at least 5 mm in length in each sample were measured by using a fine scale steel ruler. The mean length of the fragments for each sample was then calculated. The mean length of the fragments for each trial was then calculated. The results are shown in Figures 15 to 17.

### Results

The numerical results are shown in tables in Figures 15 to 17 for Trials 1 to 3, respectively. In Trial 1, the mean length of fragments of at least 5 mm for the *in vitro Sphagnum* was 10.7 mm. In Trial 2, the mean length of fragments of at least 5 mm for the first generation *in vivo Sphagnum* was 8.3 mm. In Trial 3, the mean length of fragments of at least 5 mm for the second generation *in vivo Sphagnum* was 15.7 mm.

### EXAMPLE 5

### Diameters of stems of Sphagnum

### Materials and Methods

Trial 1 was *in vitro Sphagnum* taken immediately from the laboratory after 4 months of growth. Trial 2 was first generation *in vivo Sphagnum* harvested after 6 months of growth in a heated indoor greenhouse, where *in vitro Sphagnum* was applied to produce the *in vivo Sphagnum.* Trial 3 was second generation *in vivo Sphagnum* harvested after 6 months of growth in a heated indoor greenhouse, where the first generation *in vivo Sphagnum* was harvested after 6 months of growth in a heated indoor greenhouse and applied to produce the second generation *in vivo Sphagnum.* Samples of approximately 100 g of *Sphagnum* were harvested. Ten sample strands were selected at random from these for each of the trials.

The diameter of the stems of the strands in each sample were measured. The stem diameter is measured as the thickness of the stem, preferably perpendicular to the length of the stem. A microscope was used to measure the diameter of the stem in pixels, and this was converted into a diameter is mm. The microscope used has a standard magnification of 449 pixels per mm. In other cases, such as for larger diameters, this can be measured by using a length measuring tool such as callipers. Three measurements were taken over the length of each strand. The mean diameter for each sample was then calculated by summing the measurements and dividing by the number of measurements (three).

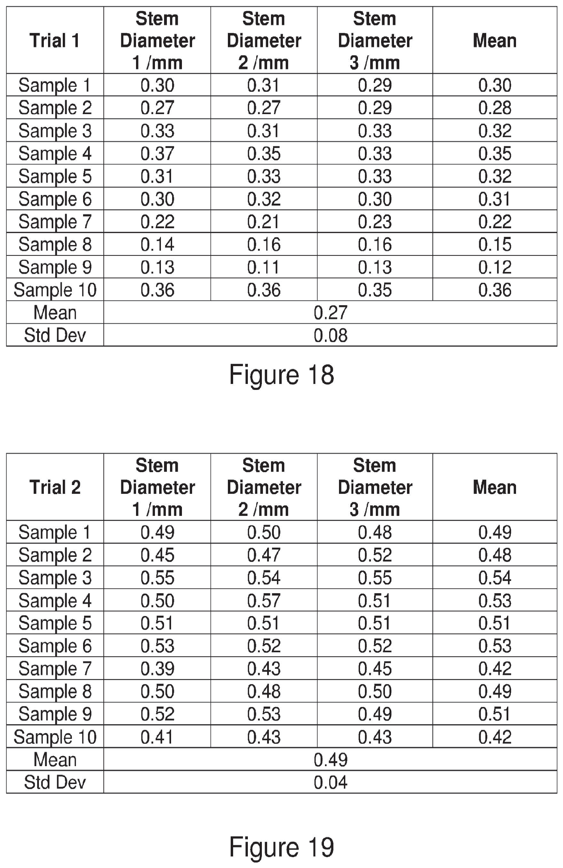

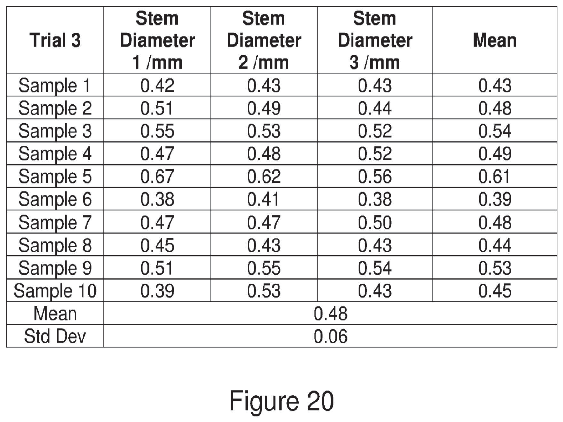

The mean diameter of the stems for each trial was then calculated. The mean can thus be calculated by summing the average diameter of each sample and dividing the total sum by the number of samples (ten). The results are shown in Figures 18 to 20.

### Results

The numerical results are shown in tables in Figures 18 to 20 for Trials 1 to 3, respectively. In Trial 1, the mean diameter of fragments for the *in vitro Sphagnum* was 0.27 mm. In Trial 2, the mean diameter of fragments for the first generation *in vivo Sphagnum* was 0.49 mm. In Trial 3, the mean diameter of fragments for the second generation *in vivo Sphagnum was* 0.48 mm.

The mean diameter increased significantly between the *in vitro Sphagnum* and the first generation *in vivo Sphagnum.* This demonstrates the rapid increase is stem thickness due to cultivating *in vivo.* The increase in stem thickness produces a much more robust fragment that is more adapted for water and nutrient uptake and water holding capacity, and provides for more effective initial establishment.

### EXAMPLE 6

### Weight of fragments of Sphagnum

### Materials and Methods

Trial 1 was *in vitro Sphagnum* taken immediately from the laboratory after 4 months of growth. Trial 2 was first generation *in vivo Sphagnum* harvested after 6 months of growth in a heated indoor greenhouse, where *in vitro Sphagnum* was applied to produce the *in vivo Sphagnum.* Trial 3 was second generation *in vivo Sphagnum* harvested after 6 months of growth in a heated indoor greenhouse, where the first generation *in vivo Sphagnum* was harvested after 6 months of growth in a heated indoor greenhouse and applied to produce the second generation *in vivo Sphagnum.* Three samples were taken for each of the trials.

The wet weight of the sample of fragments was measured. The water content was standardised by compressing the *Sphagnum* to a standard mass per volume by applying a force of 16 g / cm² and allowing drainage.

The dry weight of the sample of fragments was then measured. The dry weight is defined as the weight of the *Sphagnum* when no more water can be removed. This was performed by drying the *Sphagnum* at 25 °C in a humidity of less than 50% until no further weight loss was recorded. In other examples, the *Sphagnum* can be heated at 110 °C for 24 hours.

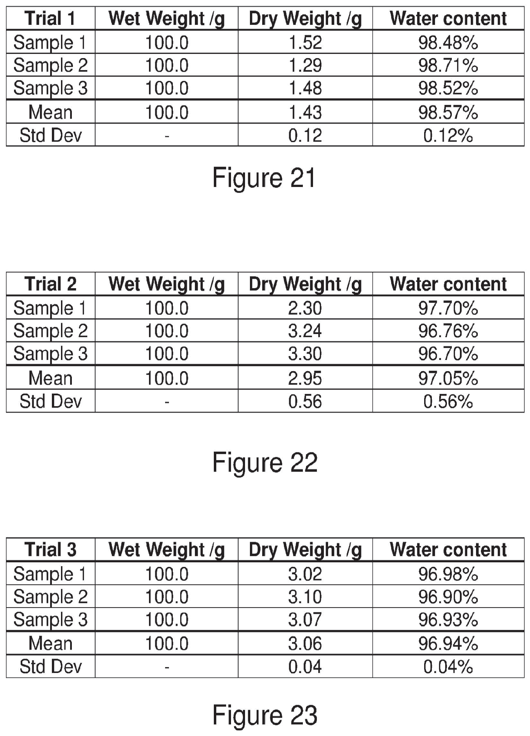

The percentage water content of the samples was then calculated by dividing the wet weight by the dry weight. The results are shown in Figures 21 to 23.

### Results

The numerical results are shown in tables in Figures 21 to 23 for Trials 1 to 3, respectively. In Trial 1, the mean dry weight of fragments for the *in vitro Sphagnum* was 1.43 g. The mean percentage water content was 98 %. In Trial 2, the mean dry weight of fragments for the first generation *in vivo Sphagnum* was 2.95 g. The mean percentage water content was 97 %. In Trial 3, the mean dry weight of fragments for the second generation *in vivo Sphagnum* was 3.06 g. The mean percentage water content was 97 %.

### EXAMPLE 7

### Distance between branches

### Materials and Methods

Each of the three trials involved samples of first generation *in vivo Sphagnum* harvested after 6 months of growth in a heated indoor greenhouse, where *in vitro Sphagnum* was applied to produce the *in vivo Sphagnum.* Each of the trials used *Sphagnum palustre.* Five sample stems were taken for each trial.

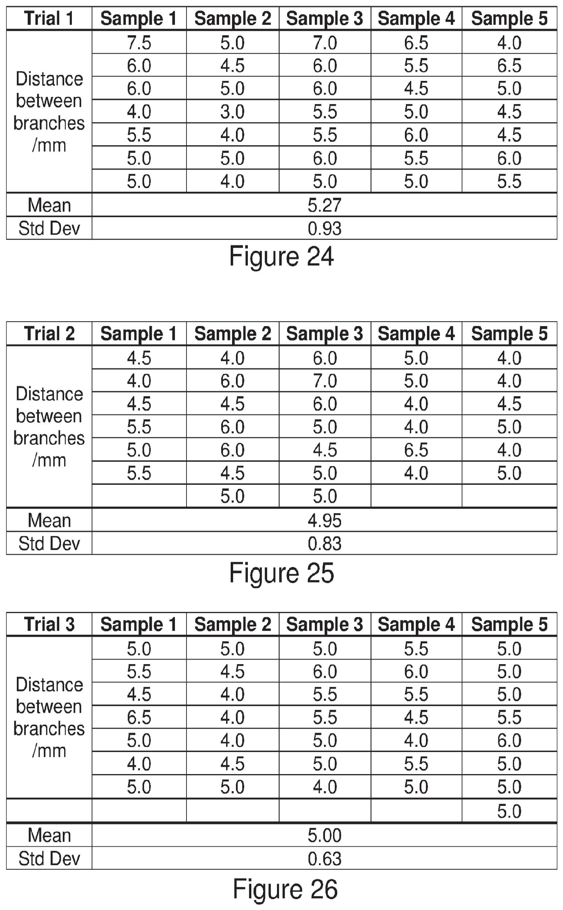

The distance between branches on each stem was measured using callipers to the nearest 0.5 mm. At least six measurements were taken on each stem. The measurements were started approximately 1 cm below the capitula. The results are shown in Figures 24 to 26.

### Results

The numerical results are shown in tables in Figures 24 to 26 for Trials 1 to 3, respectively. In Trial 1, the average distance between branches was 5.27 mm. In Trial 2, the average distance between branches was 4.95 mm. In Trial 3, the average distance between branches was 5.00 mm. Overall, the average across each trial is 5.08 mm. This supports providing fragment sizes of at least about 5 mm to provide a branch as a potential innovation.

### EXAMPLE 8

### Comparison vs wild Sphagnum - Borth

### Materials and Methods

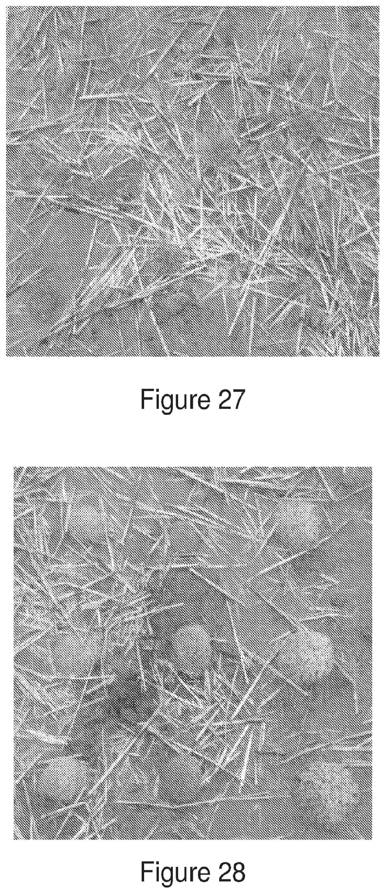

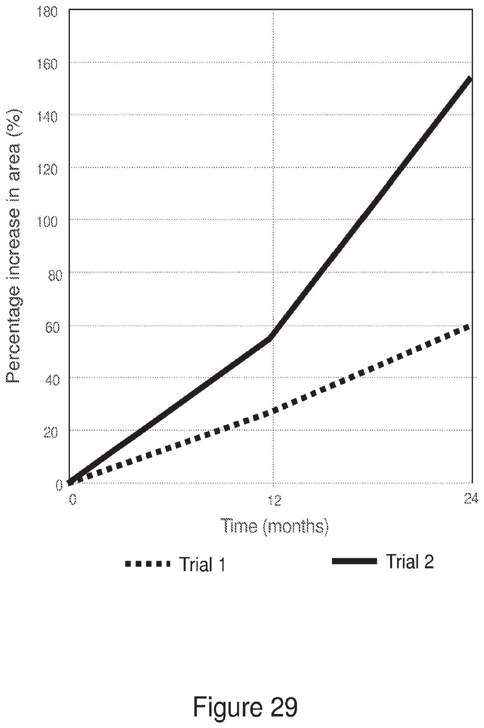

Trials were conducted at Cors Fochno, Borth, Wales. Trial 1 was wild *Sphagnum* harvested and translocated to the trial site. Trial 2 was *Sphagnum* grown in accordance with the present disclosure. In particular, Trial 2 was *Sphagnum* grown from second generation *in vivo Sphagnum.* Each trial contained three species: *S. papillosum* (left of Figures 28 and 29), *S. capillifolium* (middle), and *S. palustre* (right), and each species had three replicates. The trials were on a raised peat bog and left outdoors for 5 months. After 5 months of growth photographs were taken and the percentage growth by increase in area was calculated.

### Results

The photographs are shown in Figures 27 and 28 for Trial 1 and 2, respectively. In Trial 1, the *Sphagnum* is visually seen to have grown slowly. The percentage increase in growth was 37 %.

In Trial 2, the *Sphagnum* grew much faster and was much quicker to establish. All three species are observed to have grown successfully, and each sample grew better than Trial 1. The percentage increase in growth was 285 %. This provides over 7 times the growth rate of the wild *Sphagnum* of Trial 1.

This shows that, not only does the *Sphagnum* grown according to the present disclosure provide an improvement compared to growing *in vitro Sphagnum,* but it also provides an improvement compared to wild *Sphagnum.*

### EXAMPLE 9

### Comparison vs wild Sphagnum - Kinder Scout

### Materials and Methods

Trials were conducted at Kinder Scout, Derbyshire Peak District, England. Trial 1 was wild *Sphagnum* harvested and translocated to the trial site. Trial 2 was *Sphagnum* grown in accordance with the present disclosure. In particular, Trial 2 was *Sphagnum* grown from second generation *in vivo Sphagnum.* The trials were left outdoors for 24 months. After intervals of 12 months of growth, the percentage increase in area was measured for each trial.

### Results

The values are shown in the graph of Figure 29. In Trial 1, the percentage increase in area was constantly below that of Trial 2. After 24 months, the percentage increase in area for Trial 1 was approximately 60 %, whereas for Trial 2 it was over 150 %.

This shows that the *Sphagnum* grown according to the present disclosure provides an improvement in rapid establishment and also long term growth rates compared to wild harvested *Sphagnum.*

### EXAMPLE 10

### Viscosity properties

### Materials and Methods

Suspensions of *Sphagnum* were prepared in accordance with the present disclosure. A control was tested, which contained the suspension without the *Sphagnum.* Trial 1 and Trial 2 were the same suspension, with approximately 100g per L of *Sphagnum* in the suspension. The suspensions comprised a fluid solution of a thickening agent of hydroxyethyl cellulose dissolved in water. The thickening agent was Natrosol^{™} HHX present at 7.25 g per L. Trial 2 was the same suspension as Trial 1, but obtained after several hours after initial mixing to test any temperature fluctuation effects.

A tray was used to observe the speed of horizontal flow of the suspensions. The tray had an internal length of 34 cm and a width of 27 cm. The tray was divided into a holding region and a flowing region by a divider in the form of a polystyrene pad extending across a width of the tray. The holding region was defined as the region between the divider and the end of the tray. The holding region had an area of approximately 192 cm². A finish line was marked on the tray at a distance of 15 cm from the holding region.

The tray was placed on a level surface, which was ensured by use of a spirit level. For each trial, 500 ml of the suspension was placed into the holding region of the tray. The temperature of the suspension was measured to be 14 °C across each trial. The divider retained the suspension in the holding region. The divider was then raised to allow the suspension to flow freely across the tray. A timer was immediately started when the divider was raised. The timer was then stopped when the leading edge flow line of the suspension had entirely crossed the finish line. The time elapsed was then recorded.

The suspension was then removed from the tray, and the tray was replaced back onto the level surface and the divider lowered into position. This process was repeated three times with different samples of the same suspension to provide four sample times for each suspension. An average of the four samples was then calculated.

### Results

The time for the suspension to flow out of the holding region and across the finish line provides an indication of the viscous properties of the suspension. The more viscous the suspension, the longer it will take for the suspension to cross the finish line.

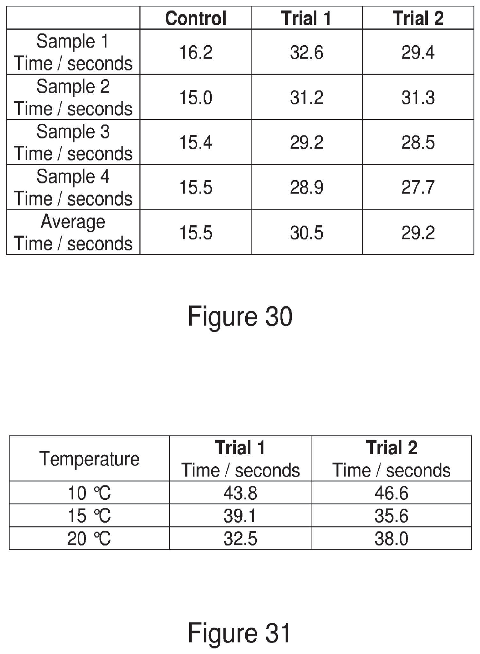

The values are shown in the table of Figure 30. Control provided an average time of 15.5 seconds. Trial 1 provided an average time of 30.5 seconds. This shows the *Sphagnum* significantly increases the viscosity of the suspension. This also provides a measure of the desired viscosity of a preferred embodiment of the present disclosure. Trial 2 provided an average time of 29.2 seconds, consistent with Trial 1.

### EXAMPLE 11

### Viscosity - temperature effect

### Materials and Methods

Suspensions of *Sphagnum* were prepared in accordance with the present disclosure. Trial 1 and Trial 2 were suspensions comprising a fluid solution of a thickening agent of hydroxyethyl cellulose dissolved in water. Trial 1 contained a thickening agent of Natrosol^{™} HHX present at 7.25 g per L. Trial 2 was the same suspension as Trial 1, but instead contained a thickening agent of Natrosol^{™} HX present at 9 g per L. Each trial was tested at different room temperatures including 10 °C, 15 °C, and 20 °C.

Each of the trials were then subject to the timing experiment of Example 10.

### Results

The values are shown in the table of Figure 31. Trial 1 provided a time of 43.8 seconds at 10 °C, 39.1 seconds at 15 °C, and 32.5 °C at 20 °C. Trial 2 provided a time of 46.6 seconds at 10 °C, 35.6 seconds at 15 °C, and 38.0 seconds at 20 °C. This shows that the time between the different compositions (i.e. between different thickening agents) can be similar for different temperatures. In other words, similar viscosity properties can be achieved through appropriate selection of concentration of thickening agent.

Further aspects of the present disclosure are set out in the following clauses:
1. A suspension of *Sphagnum,* comprising:
   a fluid solution comprising:
      water; and
      a thickening agent dissolved in the water, wherein the thickening agent comprises a cellulose-based or a starch-based thickening agent; and
   a plurality of fragments of *Sphagnum* suspended in the fluid solution.
2. The suspension according to clause 1, wherein the thickening agent comprises a cellulose ether.
3. The suspension according to clause 1 or 2, wherein the thickening agent comprises hydroxyethyl cellulose.
4. The suspension according to clause 3, wherein the fluid solution comprises between 5 g and 10 g of hydroxyethyl cellulose per L of water.
5. The suspension according to any preceding clause, wherein the thickening agent comprises an extract from a plant.
6. The suspension according to any preceding clause, wherein the thickening agent does not comprise algin.
7. The suspension according to any preceding clause, wherein the thickening agent does not comprise agar.
8. The suspension according to any preceding clause, wherein the fluid solution comprises nutrients.
9. The suspension according to clause 8, wherein the nutrients comprise calcium.
10. The suspension according to clause 9, wherein the nutrients comprise between 1 mg and 50 mg of calcium per L of water.
11. The suspension according to any of clauses 8 to 10, wherein the nutrients comprise at least one of: magnesium, nitrogen, potassium, and/or phosphorus.
12. The suspension according to any preceding clause, wherein the fluid solution does not solidify for at least 6 hours at a temperature between 5 °C and 25 °C.
13. The suspension according to any preceding clause, wherein the suspension is adhesive to a growing substrate comprising soil, sand, compost, peat and/or dried *Sphagnum.*
14. The suspension according to any preceding clause, wherein the suspension provides capillary contact with a surface to which it is applied to enable fluid transfer between the surface and the suspension.
15. The suspension according to any preceding clause, wherein the suspension is capable of being sprayed through a nozzle having a diameter of between 5 mm and 10 mm.
16. The suspension according to any preceding clause, wherein the fluid solution has a viscosity of between 1000 mPa·s and 4000 mPa·s at 25 °C.
17. The suspension according to any preceding clause, wherein the fragments of *Sphagnum* are cultivated *in vitro.*
18. The suspension according to clause 17, wherein the fragments of *Sphagnum* have subsequently been cultivated *in vivo.*
19. The suspension according to any preceding clause, wherein the fragments of *Sphagnum* have a mean length of between 5 mm and 50 mm.
20. The suspension according to any preceding clause, wherein at least 50% by mass of the fragments of *Sphagnum* have a length of at least 5 mm.
21. The suspension according to any preceding clause, wherein the suspension comprises at least 1000 fragments of *Sphagnum* having a length of at least 5 mm per L of fluid solution.
22. The suspension according to any preceding clause, wherein the suspension comprises a total mass of fragments of *Sphagnum* of at least 50 g per L of fluid solution.
23. The suspension according to any preceding clause, wherein the fragments of *Sphagnum* have a mean stem diameter of between 0.1 mm and 1 mm.
24. A method of producing a suspension of *Sphagnum,* the method comprising:
   providing a plurality of fragments of *Sphagnum;*
   preparing a fluid solution comprising:
      providing water; and
      dissolving a thickening agent in the water, wherein the thickening agent comprises a cellulose-based or a starch-based thickening agent; and
   mixing the plurality of fragments of *Sphagnum* with the fluid solution to suspend the plurality of fragments of *Sphagnum* in the fluid solution.
25. The method according to clause 24, wherein the suspension comprises the suspension according to any of clauses 1 to 23.

### CLAUSES:

1. A method of providing a seedstock of *Sphagnum,* comprising:
   providing *in vitro Sphagnum;*
   applying the *Sphagnum* to a growth surface;
   cultivating the *Sphagnum in vivo* on the growth surface;
   harvesting the cultivated *Sphagnum* from the growth surface; and
   chopping the harvested *Sphagnum* to provide a seedstock of *Sphagnum* for cultivation, the seedstock comprising a plurality of fragments of the *in vivo Sphagnum.*
2. The method according to clause 1, wherein the fragments of *in vivo Sphagnum* have a mean stem diameter which is at least 50% thicker than a mean stem diameter of the *in vitro Sphagnum.*
3. The method according to clause 1 or 2, wherein the fragments of *in vivo Sphagnum* have a mean stem diameter of at least 0.4 mm, and wherein the *in vitro Sphagnum* has a mean stem diameter of less than 0.3 mm.
4. The method according to any preceding clause, wherein the fragments of *in vivo Sphagnum* have a mean length of between 5 and 50 mm.
5. The method according to any preceding clause, wherein the providing *in vitro Sphagnum* comprises chopping *in vitro Sphagnum* into a plurality of fragments of *in vitro Sphagnum.*
6. The method according to clause 5, wherein the providing *in vitro Sphagnum* comprises mixing the plurality of fragments of *in vitro Sphagnum* with a first fluid solution to provide a suspension of *in vitro Sphagnum.*
7. The method according to clause 6, wherein the first fluid solution comprises water and a thickening agent dissolved in the water.
8. The method according to clause 6 or 7, wherein the suspension of *in vitro Sphagnum* comprises at least 50 g of *in vitro Sphagnum* per L of the first fluid solution.
9. The method according to any of clauses 6 to 8, wherein the applying comprises applying the suspension of *in vitro Sphagnum* onto the growth surface at a density of between 0.5 and 3 L / m².
10. The method according to any of clauses 6 to 9, wherein the applying comprises spraying the suspension of *in vitro Sphagnum* onto the growth surface.
11. The method according to clause 10, wherein the spraying comprises pumping the suspension of *in vitro Sphagnum* through an aperture to hit a deflecting plate.
12. The method according to any preceding clause, wherein the growth surface comprises compost, soil, sand, peat, and/or dried *Sphagnum.*
13. The method according to any preceding clause, wherein the cultivating is carried out under controlled environmental conditions, wherein the controlled environmental conditions comprise at least one of: irrigation, nutrient supply, lighting, shading, temperature, and/or humidity.
14. The method according to any preceding clause, wherein the cultivating comprises irrigating the *Sphagnum,* comprising applying water at a rate of at least 1 L / m² / day.
15. The method according to any preceding clause, wherein the cultivating comprises applying nutrients to the *Sphagnum.*
16. The method according to any preceding clause, wherein the cultivating is carried out for at least one month.
17. The method according to any preceding clause, wherein the chopping the harvested *Sphagnum* comprises chopping such that at least 50 % by weight of the fragments of *in vivo Sphagnum* have a length of at least 5 mm.
18. The method according to any preceding clause, wherein the providing the seedstock of *Sphagnum* comprises mixing the plurality of fragments of *in vivo Sphagnum* with a second fluid solution to provide a suspension of *in vivo Sphagnum.*
19. The method according to clause 18, wherein the second fluid solution comprises water amd a tickening agent dissolved in the water.
20. The method according to clause 19, wherein the thickening agent is a cellulose-based or starch-based thickening agent.
21. The method according to clause 20, wherein the thickening agent comprises hydroxyethyl cellulose.
22. The method according to clause 21, wherein the second fluid solution comprises between 5 g and 10 g of hydroxyethyl cellulose per L of water.
23. A method comprising:
   performing the method according to any preceding clause;
   and applying the seedstock of *Sphagnum* to a growth
      surface.
24. The method according to clause 23, further comprising cultivating the *Sphagnum* of the seedstock *in vivo* on the growth surface.
25. The method according to clause 24, further comprising harvesting the *Sphagnum* of the seedstock from the growth surface.
26. The method according to clause 25, further comprising providing a growing medium comprising the harvested *Sphagnum* of the seedstock, comprising drying the harvested *Sphagnum* of the seedstock.
27. The method according to clause 25, further comprising chopping the harvested *Sphagnum* from the seedstock to provide a second seedstock of *Sphagnum* for cultivation, the second seedstock comprising a plurality of fragments of second generation *in vivo Sphagnum.*
28. The method according to clause 27, wherein the providing the second seedstock of *Sphagnum* comprises mixing the plurality of fragments of second generation *in vivo Sphagnum* with a third fluid solution to provide a suspension of *in vivo Sphagnum.*
29. The method according to clause 28, wherein the third fluid solution comprises water and a thickening agent dissolved in the water.
30. The method according to clause 29, wherein the thickening agent is a cellulose-based or starch-based thickening agent.
31. The method according to clause 30, wherein the thickening agent comprises hydroxyethyl cellulose.
32. The method according to clause 31, wherein the third fluid solution comprises between 5 g and 10 g of hydroxyethyl cellulose per L of water.
33. A seedstock of *Sphagnum* obtainable by the method according to any of clauses 1 to 22.
*34. Sphagnum* obtainable by the method according to clause 24 or 25.
35. A seedstock of *Sphagnum* obtainable by the method according to any of clauses 27 to 32.
36. A seedstock of *Sphagnum* comprising:
   a plurality of fragments of *Sphagnum*;
   wherein at least 25 % of the fragments of *Sphagnum* have a length of between 5 mm and 50 mm; and
   wherein at least 25 % of the fragments of *Sphagnum* have a mean stem diameter of at least 0.4 mm.

## Claims

1. A suspension of *Sphagnum,* comprising:
a fluid solution comprising:
water; and
a thickening agent dissolved in the water, wherein the thickening agent comprises a cellulose-based or a starch-based thickening agent; and
a plurality of fragments of *Sphagnum* suspended in the fluid solution.

2. The suspension according to claim 1, wherein the thickening agent comprises hydroxyethyl cellulose.

3. The suspension according to claim 2, wherein the fluid solution comprises between 5 g and 10 g of hydroxyethyl cellulose per L of water.

4. The suspension according to any preceding claim, wherein the thickening agent does not comprise algin and/or wherein the thickening agent does not comprise agar.

5. The suspension according to any preceding claim, wherein the fluid solution comprises nutrients, optionally wherein the nutrients comprise at least one of: calcium, magnesium, nitrogen, potassium, and/or phosphorus.

6. The suspension according to any preceding claim, wherein the fluid solution does not solidify for at least 6 hours at a temperature between 5 °C and 25 °C.

7. The suspension according to any preceding claim, wherein the suspension is capable of being sprayed through a nozzle having a diameter of between 5 mm and 10 mm and/or wherein the fluid solution has a viscosity of between 1000 mPa·s and 4000 mPa·s at 25 °C.

8. The suspension according to any preceding claim, wherein the fragments of *Sphagnum* are cultivated *in vitro,* optionally wherein the fragments of *Sphagnum* have subsequently been cultivated *in vivo.*

9. The suspension according to any preceding claim, wherein the fragments of *Sphagnum* have a mean length of between 5 mm and 50 mm.

10. The suspension according to any preceding claim, wherein at least 50% by mass of the fragments of *Sphagnum* have a length of at least 5 mm and/or wherein the suspension comprises at least 1000 fragments of *Sphagnum* having a length of at least 5 mm per L of fluid solution.

11. The suspension according to any preceding claim, wherein the suspension comprises a total mass of fragments of *Sphagnum* of at least 50 g per L of fluid solution and/or wherein the suspension comprises a total dry mass of fragments of *Sphagnum* of at least 1 g per L of fluid solution.

12. The suspension according to any preceding claim, wherein the fragments of *Sphagnum* have a mean stem diameter of between 0.1 mm and 1 mm.

13. A method of producing a suspension of *Sphagnum,* the method comprising:
providing a plurality of fragments of *Sphagnum*;
providing a fluid solution comprising:
water; and
a thickening agent dissolved in the water, wherein the thickening agent comprises a cellulose-based or a starch-based thickening agent; and
mixing the plurality of fragments of *Sphagnum* with the fluid solution to suspend the plurality of fragments of *Sphagnum* in the fluid solution.

14. The method of claim 13, wherein providing the fluid solution comprises preparing the fluid solution and wherein preparing the fluid solution comprises providing the water and dissolving the thickening agent in the water.

15. The method according to claim 13 or 14, wherein the suspension comprises the suspension according to any of claims 1 to 12.
